(19) Europäisches Patentamt
European Patent Office
Office européen des brevets

(11) Veröffentlichungsnummer: **0 002 283**
**B1**

(12) **EUROPÄISCHE PATENTSCHRIFT**

(45) Veröffentlichungstag der Patentschrift: **24.08.83**

(21) Anmeldenummer: **78101554.0**

(22) Anmeldetag: **04.12.78**

(51) Int. Cl.³: **C 07 D 495/10,**
A 61 K 31/445,
A 61 K 31/40 //C07D211/52,
C07D211/54, (C07D495/10,
333/00, 221/00),
(C07D495/10, 333/00,
209/00)

(54) **Substituierte 1,3-Dihydrospiro (benzo(c)thiophene), Verfahren zu deren Herstellung und diese Verbindungen zur Verwendung als Arzneimittel.**

(30) Priorität: **02.12.77 US 857177**
**05.10.78 US 948908**

(43) Veröffentlichungstag der Anmeldung:
**13.06.79 Patentblatt 79/12**

(45) Bekanntmachung des Hinweises auf die Patenterteilung:
**24.08.83 Patentblatt 83/34**

(84) Benannte Vertragsstaaten:
**BE CH DE FR GB IT LU NL SE**

(56) Entgegenhaltungen:
**US - A - 3 959 475**

**Burger, Medicinal Chemistry, Part I (1970), S. 75**
**J. Pharmacol. Exptl. Therap. 89 (1947) S. 131—142**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT**
**Postfach 80 03 20**
**D-6230 Frankfurt am Main 80 (DE)**

(72) Erfinder: **Ong, Helen Hu**
**Bunker Hill Place 15**
**Whippany New Jersey (US)**
Erfinder: **Anderson, Vernon Brian**
**Sylvan Road 24**
**High Bridge New Jersey (US)**
Erfinder: **Profitt, James Arthur**
**Robbins Road 62**
**Somerville New Jersey (US)**

**EP 0 002 283 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

Substituierte 1,3-Dihydrospiro[benzo(c)thiophene], Verfahren zu deren Herstellung und diese Verbindungen zur Verwendung aels Arzneimittel.

Vorliegende Erfindung betrifft neue substituierte 1,3-Dihydrospiro[benzo(c)thiophene], welche ale Antidepressiva, Beruhigungsmittel sowie als Zwischenprodukte dafür nützlich sind, Verfahren zu deren Herstellung, sowie solche Verbindungen als wesentliche Wirkstoffe enthaltende Arzneimittel.

Nach unserem besten Wissen sind die erfindungsgemässen Verbindungen bisher weder beschrieben noch vorgeschlagen worden. Substituierte 1,3-Dihydrospiro[isobenzofurane] der Formel

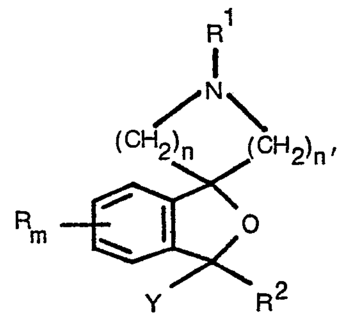

worin R für Wasserstoff, Alkyl, Alkoxy, Trifluormethyl, Halogen, Hydroxyl oder Methylendioxy, $R^1$ für Wasserstoff, Alkyl, Cycloalkylalkyl, Alkenyl, Phenylalkyl, Diphenylalkyl, Diphenylmethoxyalkyl, Alkanoyl, Phenylalkanoyl, Benzoyl, Benzoylalkyl, Phenylhydroxyalkyl, Alkoxycarbonyl, Phenoxycarbonyl oder Cycloalkylcarbonyl, $R^2$ für Alkyl oder Phenyl und Y für Wasserstoff, Alkyl, Alkoxy oder Hydroxyl steht und m, n und n' ganze Zahlen von 1 bis 3 darstellen, sowie deren optische Antipoden und pharmazeutisch unbedenkliche Säureadditionssalze sind von Victor J. Bauer u.a. in dem am 25. Mai 1976 erteilten US Patent Nr. 3 959 475 beschrieben. Ferner beschreiben Marxer u.a. in "Spiro Piperidines. I. Synthesis of Spiro[isobenzofuran-1(3H),4'-piperidines] and Spiro[isobenzofuran-1(3H),3-piperidines]", J. Org. Chem., Band 40, Nr. *10* (1975), verschiedene durch die folgenden Formeln dargestellte Isobenzofurane:

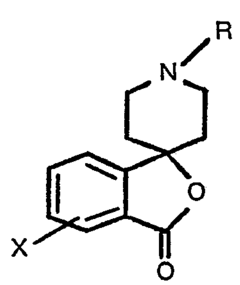

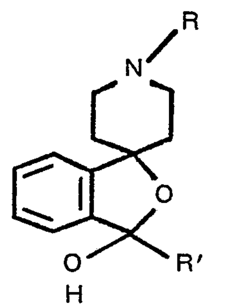

und

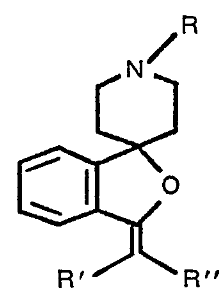

worin X für Wasserstoff, Halogen oder Methoxy, R für Methyl oder Benzyl, R' für Phenyl, substituiertes Phenyl, Alkyl, Phenylalkyl, Diphenylalkyl oder Thienyl und R'' für Alkyl, Phenyl, Benzyl, 3-Methyl-5-isoxazolyl oder Pyridyl steht. In diesem Artikel wird jedoch keine pharmazeutische Wirksamkeit der offenbarten Isobenzofurane beschrieben.

Keines dieser zwei Zitate beschreibt oder schlägt die hierin beanspruchten Verbindungen vor. Dort sind Isobenzofurane, nicht Benzo(c)thiophene offenbart. Ferner können die Verbindungen dieser Erfindung nicht durch die in dem oben erwähnten Stand der Technik beschriebenen Synthesen hergestellt werden.

Gegenstand dieser Erfindung sind neue substituierte 1,3-Dihydrospiro[benzo(c)thiophene] der Formel:

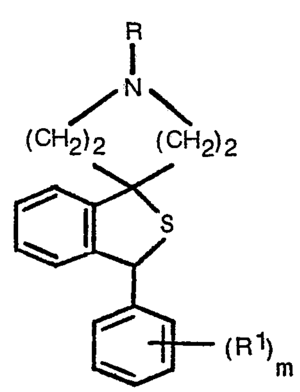

und deren optische Antipoden und pharmazeutisch unbedenklichen Salze, worin R für Wasserstoff, $C_{1-6}$-Alkyl, $C_{3-6}$-Cycloalkyl-$C_{1-6}$-alkyl oder Benzoyl-$C_{1-6}$-alkyl, wobei der Phenylrest mit Fluor substituiert sein kann, $R^1$ für Wasserstoff, $C_{1-6}$-Alkyl oder Halogen steht und m 1 oder 2 bedeutet.

Bei der Herstellung der erfindungsgemäßen pharmazeutisch unbedenklichen Säureadditionssalze verwendbare Säuren sind unter anderem anorganische Säuren wie Salz-, Bromwasserstoff-, Schwefel, Salpeter-, Phosphor- und Ueberchlorsäure sowie organische Säuren wie Wein-, Zitronen-, Essig-, Bernstein-, Malein-, Fumar- und Oxalsäure.

Einige in den Rahmen dieser Erfindung fallende Verbindungen besitzen stärkere pharmazeutische Wirksamkeit als andere. Die letzteren Verbindungen sind trotzdem als Zwischenprodukte bei der Herstellung der wirksameren Verbindungen erwünscht und nützlich. Bevorzugte Verbindungen sind solche, worin R für Wasserstoff, Alkyl mit 1 bis 3 Kohlenstoffatomen, insbesondere Methyl, oder Fluor-substituiertes Benzoylpropyl steht.

Die erfindungsgemäßen Verbindungen lassen sich nach einer der unten beschriebenen Herstellungsmethoden erzeugen. Mit den angegebenen Ausnahmen besitzen R, $R^1$, $R^2$, n und m in dieser Beschreibung die zuvor angegebenen Bedeutungen.

Methode A

Man überführt ein 2-Bromfluorbenzol nach einer zweckmäßigen Methode, z.B. durch Umsetzung mit einem Niederalkyllithium bei einer Temperatur von −100 bis −50°C in einem Lösungsmittel wie Äther, Hexan oder Tetrahydrofuran, in sein Lithioderivat. Bei einer bevorzugten Methode wird die Umsetzung mit Butyllithium in Tetrahydrofuran bei einer Temperatur zwischen −70 und −60°C, durchgeführt.

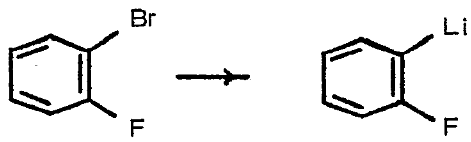

I II

Das so erhaltene 2-Lithioderivat setzt man unter üblicherweise für diese Art Umsetzung angewandten Reaktionsbedingungen, z.B. bei einer Temperatur von −80 bis −20°C, vorzugsweise −80 bis −40°C, in einem Lösungsmittel wie Äther, Tetrahydrofuran oder Hexan mit einem Cycloazalkanon der Formel

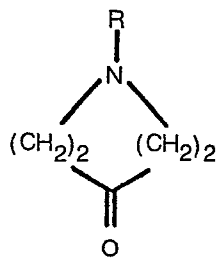

worin R für $C_{1-6}$-Alkyl steht, zu einem Phenylcycloazalkanol (III) um.

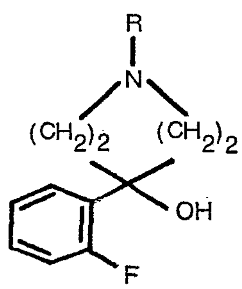

III

Das Phenylcycloazalkanol (III) behandelt man bei Raumtemperatur bis 100°C mit einem Benzylmercaptan der Formel

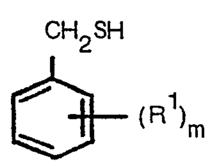

und einer Lewissäure wie Titantetrachlorid oder Bortrifluordiätherat, was das entsprechende Benzylthiophenylcycloazalkan (IV) liefert.

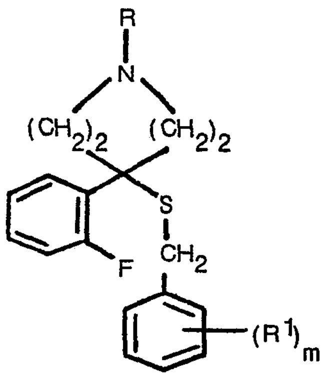

Das Benzylthiophenylcycloazalkan wird zum Ring geschlossen, was das entsprechende 1,3-dihydrospiro[benzo(c)thiophencycloazalkan] (V), eine erfindungsgemäße Verbindung, liefert.

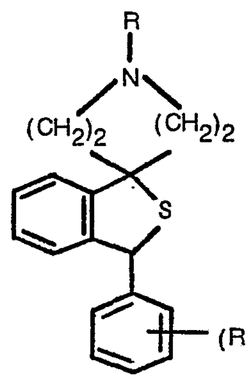

V

Bei einer bevorzugten Methode wird Natriumhydrid als Kondensations/Ringschlußmittel in einem Lösungsmittel wie Dimethylformamid bei einer Temperatur von 25 bis 100°C, angewendet.

Methode B

Man behandelt ein nach Methode A hergestelltes 1,3-Dihydrospiro[benzo(c)thiophen-cycloazalkan] (I) bei 15 bis 125°C in einem Lösungsmittel wie Toluol, Benzol oder Methylenchlorid, gegebenenfalls in Gegenwart eines säurebindenden Mittels wie Natriumbicarbonat, mit einem Chlorameisensäureester, z.B. einem Chlorameisensäurealkyl- oder -phenylester, was das entsprechende N-Alkoxycarbonyl- oder N-Phenoxy-carbonyl-1,3-dihydrospiro[benzo(c)thiophen-cycloazalkan] (VI), eine erfindungsgemäße Verbindung, worin $R^2$ für $C_{1-6}$-Alkyl oder Phenyl steht, liefert.

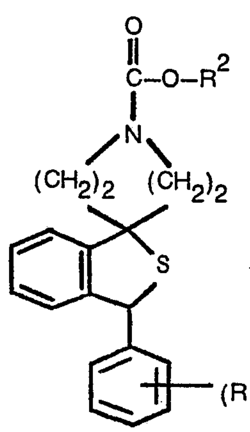

VI

Methode C

Man behandelt eine erfindungsgemäße, nach Methode B hergestelle Verbindung in einem Lösungsmittel wie Wasser, Äthanol oder Äthylenglykol bei 15°C bis Rückflußtemperatur des

Reaktionsgemisches mit einer Base wie Natrium- oder Kaliumhydroxyd oder bei einer Temperatur von 15 bis 125°C mit einer Säure wie Bromwasserstoff in Essigsäure, was das entsprechende N-unsubstituierte 1,3-Dihydrospiro[benzo(c)thiophen-cycloazalkan] (VII), eine erfindungsgemäße Verbindung, liefert.

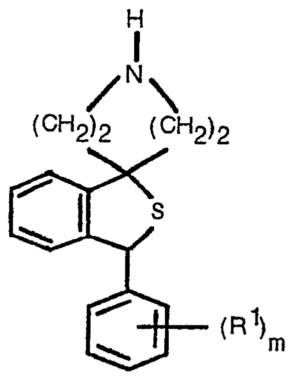

VII

Methode D

Eine nach Methode C hergestellte Verbindung kann man unter dan dabei normalen Reaktionsbedingungen mit einer Verbindung der Formel

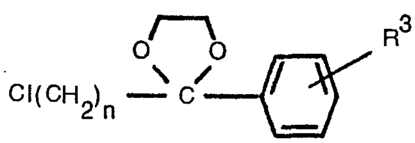

worin $R^3$ Wasserstoff oder Fluor bedeutet und n eine Zahl von 1—6 darstellt, behandeln, was die entsprechende Verbindung (VIII) liefert.

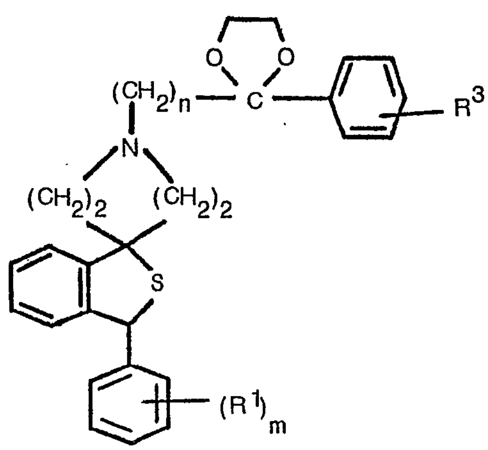

VIII

In einer bevorzugten Methode verwendet man dabei Kaliumjodid als Reaktionsinitiator, Natriumbicarbonat als säurebindendes Mittel und Dimethylformamid als Lösungsmittel be einer Reaktionstemperatur von etwa 75°C.

Methode E

Die nach Methode D hergestellte Verbindung unterwirft man einer Hydrolyse zur entsprechenden erfindungsgemäßen Verbindung, in der R

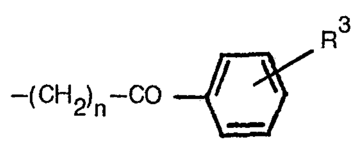

darstellt, wobei n sowie $R^3$ die oben angegebene Bedeutung haben. Bei einer bevorzugten Methode handelt es sich um die Anwendung einer starken Säure wie 3n-HCl in einem Lösungsmittel wie Wasser oder Äthanol.

Methode F

Ein nach Methode C hergestelltes, N-unsubstituiertes 1,3-Dihydrospiro[benzo(c)thiophen-cycloazalkan] (VII) kann man in bekannter Weise mit einem $C_{1-6}$-Alkanoylchlorid oder -anhydrid, $C_{1-6}$-Alkylhalogenid, $C_{3-6}$-Cycloalkyl-$C_{1-6}$-alkylhalogenid oder $C_{3-6}$-Cycloalkyl-$C_{1-6}$-alkanoylhalogenid zur entsprechenden erfindungsgemäßen Verbindung, in der R $C_{1-6}$-Alkanoyl, $C_{1-6}$-Alkyl, $C_{3-6}$-Cycloalkyl-$C_{1-6}$-alkanoyl oder $C_{3-6}$-Cycloalkyl-$C_{1-6}$-alkyl darstellt, umsetzen.

Methode G

Eine nach Methode B, E oder F hergestellte Verbindung, in der R $C_{2-6}$-Alkoxycarbonyl, $C_{1-6}$-Alkanoyl, $C_{3-6}$-Cycloalkyl-$C_{1-6}$-alkanoyl darstellt, kann man in bekannter Weise mit einem Reagenz wie Lithiumaluminiumhydrid oder Diboran zur entsprechenden erfindungsgemäßen Verbindung, in der R $C_{1-6}$-Alkyl oder $C_{3-6}$-Cycloalkyl-$C_{1-6}$-alkyl darstellt, reduzieren.

Die erfindungsgemäßen Verbindungen sind zur Behandlung von Depressionen in Säugetieren wertvoll, wie ihre Fähigkeit, an der Maus die durch Tetrabenazin induzierte Depression [International Journal of Neuropharmacology, 8, 73 (1969)], ein Standardtest auf nützliche antidepressive Eigenschaften, zu hemmen, zeigt. So bewirkt 1,3-Dihydro-3-phenylspiro[benzo(c)thiophen-1,4'-piperidin] bzw. 1,3-Dihydro-1'-methyl-3-phenylspiro[benzo(c)thiophen-1,4'-piperidin] in einer intraperitonealen Dosierung von 1,1 bzw. 2,5 mg/kg eine 50 %ige Hemmung der Ptose bei durch Tetrabenazin induzierter Depression an der Maus. Diese Werte veranschaulichen, daß die Verbindungen bei Verabreichung in einer Tagesmenge im Bereich von 0,1 bis 50 mg/kg Körpergewicht als Antidepressiva bei Säugetieren wertvoll sind.

Wegen ihrer depressiven Wirkung auf das zentrale Nervensystem von Säugetieren sind die erfindungsgemäßen Verbindungen ferner als Beruhigungsmittel wertvoll. Diese Tauglichkeit zeigt sich bei dem Sidman Avoidance Paradigm [Science, 118, 157—8 (1953)], einem Standardtest für Beruhigungsmittel, wonach sich erfindungsgemäße Verbindungen bei Verabreichung in Tagesmengen im Bereich von 0,1 bis etwa 50 mg/kg als wertvolle Beruhigungsmittel erweisen.

In der US-Patentschrift 3 959 475 sind analoge Verbindungen beschrieben, die sich von den erfindungsgemäßen Verbindungen durch den Ersatz des Ringschwefelatoms durch ein Sauerstoffatom unterscheiden. Sie haben ähnlich wie die erfindungsgemäßen Verbindungen antidepressive und beruhigende Wirkung, zeigen jedoch in stärkerem Maße als unerwünschten Nebeneffekt eine anticholinergische Wirkung.

Wirksame Mengen der erfindungsgemäßen Verbindungen können einem Patienten nach irgendeiner von verschiedenen Methoden verabreicht werden beispielsweise peroral wie in Kapseln oder Tabletten, parenteral in Form steriler Lösungen oder Suspensionen sowie in einigen Fällen intravenös in Form steriler Lösungen. Die Endprodukte selbst sind zwar als freie Basen wirksam, doch kann man sie aus Gründen der Stabilität, Kristallisierbarkeit, erhöhter Löslichkeit und dergleichen in Form ihrer pharmazeutisch unbedenklichen Additionssalze formulieren und verabreichen.

Die erfindungsgemäßen Wirkstoffe lassen sich peroral verabreichen, beispielsweise mit einem inerten Streckmittel oder essbaren Träger, oder sie können in Gelatinekapseln eingeschlossen oder zu Tabletten gepreßt werden. Zur peroralen therapeutischen Verabreichung kann man die erfindungsgemäßen Wirkstoffe in Vehikel einarbeiten und in Form von Tabletten, Pastillen, Kapseln, Elixieren, Supensionen, Sirupen, Oblaten, Kaugummi und dergleichen anwenden. Diese Präparate sollen mindestens 0,5% Wirkstoff enthalten, doch läßt sich dies in Abhängigkeit von der jeweiligen Form variieren und kann zweckmäßig zwischen 4% und etwa 70% des Einheitsgewichtes liegen. Eine solche Wirkstoffmenge liegt in derartigen Zusammensetzungen vor, daß sich eine geeignete Dosis ergibt. Bevorzugte Zusammensetzungen und Präparate gemäß vorliegender Erfindung werden so zubreitet, daß eine perorale Dosiereinheitsform 1,0—300 mg Wirkstoff enthält.

Die Tabletten, Pillen, Kapseln, Pastillen und dergleichen können ferner folgende Bestandteile enthalten: ein Bindemittel wie mikrokristalline Cellulose, Traganthgummi oder Gelatine; ferner ein Vehikel wie Stärke oder Milchzucker, ein Sprengmittel wie Alginsäure, Primogel, Maisstärke und dergleichen; ein Schmiermittel wie Magnesiumstearat oder Sterotex; ein Gleitmittel wie kolloidales Siliciumdioxyd; ferner kann man einen Süssstoff wie Rohrzucker oder Saccharin oder auch einen Geschmacksstoff wie Pfefferminz, Methyl-salicylat oder Orangenaroma zusetzen. Ist die Dosiereinheitsform eine Kapsel, so kann diese neben Stoffen der obigen Art einen flüssigen Träger wie ein fettes Oel enthalten. Andere Dosiereinheitsformen können weitere verschiedene Stoffe enthalten, welche die physikalische Form der Dosiereinheit modifizieren, wie beispielsweise Ueberzüge. Tabletten oder Pillen können somit mit Zucker oder Schellack überzogen oder mit erst im Darm löslichen Ueberzügen versehen werden. Ein Sirup kann neben den Wirkstoffen Rohrzucker als Süssstoff sowie gewisse Konservierungsmittel, Farbstoffe und Farben sowie Geschmacksstoffe enthalten. Bei der Bereitung dieser verschiedenen Zusammensetzungen verwendete Stoffe sollen pharmazeutisch rein und in den verwendeten Mengen nicht-toxisch sein.

Zur parenteralen therapeutischen Verabreichung können die erfindungsgemässen Wirkstoffe in eine Lösung oder Suspension eingearbeitet werden. Diese Zubereitungen sollen eine pharmazeutisch wirksame Menge, d.h. mindestens 0,1% Wirkstoff, enthalten, doch lässt sicht dies zwischen 0,5 und etwa 30% ihres Gewichts variieren. Eine solche Wirkstoffmenge leigt in derartigen Zusammensetzungen vor, dass sich eine geeignete Dosis ergibt. Bevorzugte Zusammensetzungen und Präparate gemäss vorleigender Erfindung werden so hergestellt, dass eine parenterale Dosiereinheit 0,5 bis 100 Milligram Wirkstoff enthält.

Die Lösungen oder Suspensionen können ferner folgende Bestandteile umfassen: ein steriles Verdünnungsmittel wie Wasser pro injectione, physiologische Kochsalzlösung, nichtflüchtige Oele, polyäthylenglykole, Glycerin, Propylenglykol oder sonstige synthetische Lösungsmittel; antibakterielle Mittel wie Benzylalkohol oder Methylparabens; Antioxydantien wie Ascorbinsäure oder Natriumbisulfit; Chelatbildner wie Aethylendiamintetraessigsäure; Puffer wie Acetate, Zitrate oder Phosphate sowie Mittel zur Einstellung des osmotischen Drucks wie Kochsalz oder Dextrose. Die parenterale Zubereitung kann in Ampullen, zum einmaligen Gebrauch bestimmten Spritzen oder Mehrfachdosisphiolen aus Glas oder Kunststoff eingeschmolzen werden.

In den nachfolgenden Beispielen wird die Erfindung näher erläutert:

Beispiel 1

a. Man gibt 46 ml 2,4m-n-Butyllithium im Verlauf von 15 Minuten zu einem auf −70°C gekühlten Gemisch aus 17,5 g 2-Bromfluorbenzol und 50 ml Tetrahydrofuran. Nach beendeter Zugabe rührt man 15 Minuten zwischen −60 und −70°C, um die Lithisierung zu Ende gehen zu lassen, was eine braunfarbene Lösung ergibt. Man versetzt mit einem Gemisch aus 11,3 g 1-Methyl-4-piperidon und 20 ml Tetrahydrofuran mit einer solchen Geschwindigkeit, dass die Temperatur de Reaktionsmediums unter −60°C bleibt. Nach beendeter Zugabe wird das Reaktionsgemisch bei derselben tiefen Temperatur eine Stunde gerührt, bevor man es sich auf Raumtemperatur erwärmen lässt. Man gibt Wasser dazu und lässt das zweiphasige Gemisch sich in seine organische und wässrige Phase scheiden. Man gewinnt die organische Phase, extrahiert die wässrige Phase dreimal mit Aether und vereinigt die Aetherextrakte mit der organischen Phase. Die vereinigte organische Lösung wird mit überschüssiger 2n-Salzsäure extrahiert und dann verworfen. Die saure Lösung macht man mit Ammoniumhydroxyd alkalisch, was ein schweres Oel liefert, welches man in Essigester/Aether 1:1 auflöst. Diese Lösung wird getrocknet und dann zur Trockne eingeengt, wobei ein viskoses Oel verbleibt, das beim Kühlen kristallisiert. Das feste Produkt wird aus Benzol/Hexan umkristallisiert, was Prismen, Schmelzpunkt 127—129°C, von 4-(2-Fluorphenyl)-4-hydroxy-1-methylpiperidin ergibt.

b. Man rührt das Reaktionsgemisch aus 4,5 g 4-(2-Fluorphenyl)-4-hydroxy-1-methylpiperidin, 8 ml Benzylmercaptan, 10 ml Bortrifluoridätherat und 10 ml Eisessig 16 Stunden bei 65°C. Die überschüssigen Reagenzien werden bei 70°C im Vakuum entfernt, und der Rückstand wird in Aether/2n-Salzsäure 1:1 aufgenommen und dann bei erniedrigter Temperatur zwei Stunden stehengelassen. Nach Abkühlung wird das kristalline Produkt zur Gewinnung abfiltriert und aus Aceton/Aether umkristallisiert, was farblose Prismen, Schmelzpunkt 182—184°C, von 4-Benzylthio-4-(2-fluorphenyl)-1-methylpiperidin-hydrochlorid ergibt.

c. Man erhitzt ein Gemisch aus 0,9 g 50%igem Natriumhydrid und 20 ml Dimethysulfoxyd in einer Stickstoffatmosphäre 30 Minuten auf 80—85°C, was eine Lösung von Natriummethylsulfinylmethid liefert, die man auf Raumtemperatur abkühlen lässt. Im Verlauf von 5 Minuten versetzt man mit der Lösung von 4,9 g 4-Benzylthio-4-(2-fluorphenyl)-1-methylpiperidin, der freien Base aus b, in 10 ml Dimethylsulfoxyd. Nach beendeter Zugabe rührt man eine Stunde bei Raumtemperatur und giesst dann auf Eiswasser. Der so erhaltene Feststoff wird durch Filtrieren gewonnen und aus Aether/Hexan umkristallisiert, was farblose Prismen, Schmelzpunkt 120—121°C, von 1,3-Dihydro-1′-methyl-3-phenylspiro[benzo(c)thiophen-1,4′-piperidin] ergibt.

Analyse:
Berechnet für $C_{19}H_{21}NS$: 77,24%C; 7,17%H; 4,74%N; 10,85%S.
Gefunden: 77,31%C; 7,20%H; 4,59%N; 10,71%S.

Beispiel 2

a. Man versetzt 5,00 g 4-(2-Fluorphenyl)-4-hydroxy-1-methylpiperidin, Beispiel la, in 11,1 ml Eisessig nacheinander mit 9,9 ml (11,9 g) 4-Chlorbenzylmercaptan und 11,1 ml Bortrifluoridätherat und rührt 48 Stunden bei 55—60°C. Die überschüssigen Reagenzien werden bei 80°C im Vakuum entfernt, und der Rückstand wird mit Aether/2n-Salzsäure 1:1 aufgenommen. Dieses zweiphasige Gemisch rührt man 2,5 Stunden und dann nach Zugabe von 50 ml Eis noch weitere zehn Minuten. Die Aetherschicht wird abgegossen. Die wässrige Schicht wird nacheinander mit Aether gewaschen und zur Gewinnung eines Rohprodukts filtriert. Dieses wäscht man mit einer kleinen Menge Wasser und dann mit einer grossen Menge Aether, was ein weisses Pulver ergibt. Dieses wird aus Aceton umkristallisiert, was weisse Kristalle, Schmelzpunkt 164—166°C, von 4-(4-Chlorbenzylthio)-4-(2-fluorphenyl)-1-methylpiperidin-hydrochlorid ergibt.

Analyse:
Berechnet für $C_{19}H_{21}NClFS.HCl$: 59,08%C; 5,74%H; 3,63%N; 4,92%F.
Gefunden: 59,04%C; 5,56%H; 3,68%N; 4,78%F.

b. Wie in Beispiel 1c bereitet man aus 0,7 g Natriumhydrid (50%) und 15 ml Dimethylsulfoxyd eine Lösung von Natriummethylsulfinylmethid. Im Verlauf von drei Minuten versetzt man mit der Lösung von 4,2 g 4-(4-Chlorbenzylthio)-4-(2-fluorphenyl)-1-methylpiperidin, der freien Base aus a, in

15 ml Diemthylsulfoxyd. Nach beendeter Zugabe rührt man eine Stunde bei Raumtemperatur und giesst dann auf 70 ml Eiswasser. Man verdünnt mit Wasser auf 120 ml Gesamtvolumen und filtriert dann zur Gewinnung des Rohprodukts. Dieses wird mit Wasser gewasschen und dann in Aether aufgelöst. Die ätherische Lösung wird nacheinander dreimal mit Wasser und einmal mit gesättigter Kochsalzlösung gewaschen und zu gelbichweissen Kristallen getrocknet. Man chromatographiert die Kristalle mit Aether durch eine Aluminiumoxydsäule, was weisse Kristalle, Schmelzpunkt 121—123°C, von 3-(4-Chlorphenyl)-1,3-dihydro-1′-methylspiro[benzo(c)thiophen-1,4′-piperidin] ergibt.
Analyse:
Berechnet für $C_{19}H_{20}ClNS$: 69,19%C; 6,11%H; 4,25%N; 10,75%Cl.
Gefunden: 69,24%C; 6,21%H; 3,96%N; 10,74%Cl.

Beispiel 3

Man rührt ein Gemisch aus 2,3 g 1,3-Dihydro-1′-methyl-3-phenylspiro[benzo(c)thiophen-1,4′-piperidin], Beispiel 1, 1,4 g Chlorameisensäurephenylester und 0,5 g Natriumbicarbonat in 40 ml Methylenchlorid 4 Stunden bei Raumtemperatur und filtriert danach. Das Filtrat wird nacheinander mit verdünnter Natronlauge und Wasser gewaschen, und getrocknet. Man entfernt das Lösungsmittel im Vakuum, wobei ein fester Rückstand verbleibt, den man aus Benzol/Hexan umkristallisiert, was Rosetten, Schmelzpunkt 171—173°C, von 1,3-Dihydro-1′-phenoxycarbonyl-3-phenylspiro[benzo(c)thiophen-1,4′-piperidin] ergibt.
Analyse:
Berechnet für $C_{25}H_{23}NO_2S$: 74,78%C; 5,77%H; 3,49%N.
Gefunden: 75,04%C; 5,84%H; 3,43%N.
Das Produkt dient zur weiteren Umsetzung.

Beispiel 4

Man rührt ein Gemisch aus 3,0 g 1,3-Dihydro-1′-phenoxycarbonyl-3-phenylspiro[benzo(c)thiophen-1,4′-piperidin], Beispiel 3, und 8,0 g Kaliumhydroxydplätzchen in 50 ml Aethylenglykol bei 155°C, bis eine klare Lösung entsteht. Man lässt abkühlen, verdünnt mit Wasser und extrahiert das zweiphasige Gemisch dreimal mit Chloroform. Die vereinigten Chloroformextrakte werden sorgfältig mit Wasser gewaschen, getrocknet und das Chloroform unter Hinterlassung eines festen Rückstands entfernt. Der Rückstand wird aus Aceton/Hexan umkristallisiert, was Prismen, Schmelzpunkt 142—144°C, von 1,3-Dihydro-3-phenylspiro[benzo(c)thiophen-1,4′-piperidin] ergibt.
Analyse:
Berechnet für $C_{18}H_{19}NS$: 76,83%C; 6,80%H; 4,98%N; 11,39%S.
Gefunden: 77,05%C; 6,84%H; 4,68%N; 11,26%S.

Beispiel 5

Man rührt ein Gemisch aus 0,6 g 1,3-Dihydro-3-phenylspiro[benzo(c)thiophen-1,4′-piperidin], Beispiel 4, 0,7 g $\gamma$-Chlor-4-fluorbutyrophenon-äthylenketal, 0,5 g Kaliumjodid und 0,5 g Natriumbicarbonat in 15 ml Dimethylformamid 16 Stunden bei 80°C. Man lässt abkühlen und verdünnt dann mit Methylenchlorid. Das verdünnte Gemisch wird filtriert und das Filtrat in Vakuum eingeengt, wobei ein Oel verbleibt, das beim Abkühlen kristallisiert. Das feste Produkt wird aus Aether/Pentan umkristallisiert, was bräunliche Kristalle, Schmelzpunkt 121—122°C, von 1′-[3-(4-Fluorbenzoyl)-propyl]-1,3-dihydro-3-phenylspiro[benzo(c)thiophen-1,4′-piperidin]-äthylenketal ergibt.
Analyse:
Berechnet für $C_{30}H_{32}FNO_2S$: 73,59%C; 6,59%H; 2,86%N.
Gefunden: 73,81%C; 6,33%H; 2,74%N.
Das Produkt dient zur weiteren Umsetzung.

Beispiel 6

Man rührt eine Probe des Oels aus Beispiel 5, 1′-[3-(4-Fluorbenzoyl)-propyl]-1,3-dihydro-3-phenylspiro[benzo(c)thiophen-1,4′-piperidin]-äthylenketal, 16 Stunden mit überschüssiger 3n-Salzsäure in Aethanol. Man entfernt den Ueberschuss Säure und Aethanol im Vakuum und nimmt den Rückstand in einem Gemisch aus 20%iger Natronlauge und Methylenchlorid auf. Man lässt das zweiphasige Gemisch sich scheiden, gewinnt und trocknet die organische Phase und entfernt das Lösungsmittel, wobei ein rötliches Oel verbleibt, das beim Abkühlen kristallisiert. Das feste Produkt wird aus Aether/Pentan umkristallisiert, was Kristalle, Schmelzpunkt 118—120°C, von 1′-[3-(4-Fluorbenzoyl)-propyl]-1,3-dihydro-3-phenylspiro[benzo(c)thiophen-1,4′-piperidin] ergibt.
Analyse:
Berechnet für $C_{28}H_{28}FNOS$: 75,47%C; 6,33%H; 3,14%N; 7,20%S.
Gefunden: 75,74%C; 6,45%H; 2.93%N; 7,21%S.

Beispiel 7

Man behandelt eine Probe 3-(4-Chlorphenyl)-1,3-dihydro-1′-methylspiro[benzo(c)thiophen-1,4′-piperidin], Beispiel 2, nach der Arbeitsweise von Beispiel 3, wobei man 3-(4-Chlorphenyl)-1,3-dihydro-1'-phenoxycarbonylspiro[benzo(c)thiophen-1,4'-piperidin] als glasigen Feststoff erhält. Dieser wird durch eine Silikagelsäule mit Methylenchlorid als Eluiermittel chromatographiert, was ein reines weisses festes Produkt vom Schmelzpunkt 211—212°C ergibt.

Analyse:

Berechnet für $C_{25}H_{22}ClNO_2S$: 68,81%C; 5,08%H; 3,21%N.

Gefunden: 69,04%C; 5,03%H; 2,97%N.

Das Produkt dient zur weiteren Umsetzung.

Beispiel 8

Man versetzt eine Lösung von 1,3-Dihydro-3-phenylspiro[benzo(c)thiophen-1,4'-piperidin], Beispiel 4, und Triäthylamin in 50 ml Chloroform kalt unter Rühren tropfenweise mit einer Lösung von Acetylchlorid in Chloroform, was 1'-Acetyl-1,3-dihydro-3-phenylspiro[benzo(c)thiophen-1,4'-piperidin] liefert.

Das Produkt dient zur weiteren Umsetzung.

Beispiel 9

Man versetzt eine Lösungs von 1'-Acetyl-1,3-dihydro-3-phenylspiro[benzo(c)thiophen-1,4'-piperidin], Beispiel 8, in Tetrahydrofuran vorsichtig unter Rühren mit einer Suspension von Lithiumaluminiumhydrid in Tetrahydrofuran, was 1'-Aethyl-1,3-dihydro-3-phenylspiro[benzo(c)thiophen-1,4'-piperidin] liefert.

Beispiel 10

a. Man versetzt die Lösung von 6,0 g 4-(2-Fluorphenyl)-4-hydroxy-1-methylpiperidin, Beispiel 1a, und 9 ml 4-Methyl-benzylmercaptan in 13,3 ml Eisessig unter gutem Rühren mit 13,3 ml Bortrifluoridätherat. Nach diesem Zusatz erhitzt man zwei Stunden bei 60—65°C. Danach wird jeglicher Ueberschuss der Reagenzien bei 70°C im Vakuum entfernt, wobei ein Rückstand verbleibt, den man mit 50 ml 2n-Salzsäure und 50 ml Aether rührt, wobei ein halbfestes Produkt in einer sich bildenden Zwischenschicht entsteht. Man gewinnt diesen halbfesten Stoff und wandelt ihn mit 20%iger Natronlauge in seine freie Base um. Diese wird in ein Hydrobromid überführt, das aus Methanol/Aether umkristallisiert wird, wobei 4-(2-Fluorphenyl)-1-methyl-4-(4-methylbenzylthio)-piperidin-hydrobromid als wiesser Feststoff vom Schmelzpunkt 182—184°C verbleibt.

b. Im Verlauf von fünf Minuten versetzt man die Lösung von 4,1 g 4-(2-Fluorphenyl)-1-methyl-4-(4-methylbenzylthio)-piperidin, der freien Base aus a, in 15 ml Dimethylsulfoxyd mit einem Gemisch aus 0,7 g Natriumhydrid (50% in Mineralöl) und 20 ml Dimethylsulfoxyd. Nach beendeter Zugabe rührt man 30 Minuten und giesst anschliessend in 100 ml Eiswasser. Das zweiphasige Gemisch wird filtriert, wobei man einen braunen Feststoff gewinnt, der in Dichlormethan gelöst und dann auf einer Aluminiumoxyd/Aethersäule mit Aether als Eluiermittel säulenchlromatographiert wird. Das chromatographierte Produkt kristallisiert man aus Aether/Hexan um, was 1,3-Dihydro-1',methyl-3-(4-methylphenyl)-spiro[benzo(c)thiophen-1,4'-piperidin] als Produkt vom Schmelzpunkt 121—122°C liefert.

Analyse:

Berechnet für $C_{20}H_{23}NS$: 77,62%C; 7,49%H; 4,53%N.

Gefunden: 77,63%C; 7,67%H; 4,35%N.

Beispiel 11

Man gibt die Lösung von 1,0 g 1,3-Dihydro-1'-methyl-3-(4-methylphenyl)-spiro[benzo(c)thiophen-1,4-piperidin], Beispiel 10, in 10 ml Dichlormethan unter Rühren zu einer Aufschlämmung von 0,6 g Chlorameisensäurephenylester und 0,2 g Natriumbicarbonat in 20 ml Dichlormethan. Nach der Zugabe lässt man 24 Stunden bei Raumtemperatur rühren, filtriert anschliessend, verdünnt mit 50 ml Methylchlorid, extrahiert mit 10%iger Natronlauge, extrahiert mit Wasser, trocknet und filtriert. Das Lösungsmittel wird entfernt, wobei ein Oel verbliebt. Dieses wird auf einer Silikagel/Methylenchloridsäule mit Methylenchlorid als Eluiermittel säulenchromatographiert, wobei man einen weissen flockigen Feststoff erhält, der aus Benzol/Hexan umkristallisiert wird, was 1,3-Dihydro-3-(4-methylphenyl)-1'-phenoxycarbonylspiro[benzo(c)thiophen-1,4'-piperidin] als Produkt vom Schmelzpunkt 179—180°C ergibt.

Analyse:

Berechnet für $C_{26}H_{25}NO_2S$: 75,15%C; 6,06%H; 3,37%N.

Gefunden: 75,24%C; 6,10%H; 3,16%N.

Das Produkt dient zur weiteren Umsetzung.

Beispiel 12

Man rührt ein Gemisch aus 0,5 g 1,3-Dihyro-3-phenylspiro[benzo(c)thiophen-1,4'-piperidin], Beispiel 4, 0,4 g Cyclopropylcarbonylchlorid und 1,0 g Natriumbicarbonat in 20 ml Dichlormethan 16 Stunden bei Raumtemperatur. Das gut gerührte Gemisch wird filtriert und das Filtrat zur Trockne eingeengt. Den Rückstand lässt man mit Aether als Eluiermittel durch eine Silikagel/Aethersäule laufen, was reines 1'-Cyclopropylcarbonyl-1,3-dihydro-3-phenylspiro[benzo(c)thiophen-1,4'-piperidin] liefert.

Analyse:
Berechnet für $C_{22}H_{23}NOS$: 75,60%C; 6,63%H; 4,01%N.
Gefunden: 75,56%C; 6,69%H; 3,71%N.
Das Produkt dient zur weiteren Umsetzung.

Beispiel 13

Man behandelt ein Gemisch aus 14,4 g 3-(4-Chlorphenyl)-1,3-dihydro-1′-phenoxycarbonyl-spiro[benzo(c)thiophen-1,4′-piperidin], Beispiel 7, 220 ml Aethylenglykol und 35,3 g Kaliumhydroxydplätzchen nach der Arbeitsweise des obigen Beispiels 4, was einen gelblichweissen kristallinen Feststoff liefert. Dieser wird mit Pentan umgeschwenkt und dann 16 Stunden bie 0°C stehengelassen, bevor man filtriert, was ein weisses Pulver liefert, das aus Aceton/Hexan umkristallisiert wird, wobei man 3-(4-Chlorphenyl)-1,3-dihydrospiro[benzo(c)thiophen-1,4′-piperidin] als Produkt vom Schmelzpunkt 138—140°C gewinnt.
Analyse:
Berechnet für $C_{18}H_{18}ClNS$: 68,46%C; 5,75%H; 4,44%N; 11,23%Cl.
Gefunden: 68,48%C; 5,81%H; 4,34%N; 11,27%Cl.

Beispiel 14

Man gibt 1,4 g Natriumbicarbonat und 1,4 g Kaliumjodid zu einem Gemisch aus 2,0 g 3-(4-Chlorphenyl)-1,3-dihydrospiro[benzo(c)thiophen-1,4′-piperidin], Beispiel 13, und 2,0 g $\gamma$-Chlor-4-fluorbutyrophenon-äthylenketal in 20 ml Dimethylformamid. Unter Rühren erhitzt man 17 Stunden auf 80—85°C. Man lässt auf Raumtemperatur abkühlen und verdünnt dann mit 50 ml Chloroform. Die verdünnte Lösung wird zur Trockne eingeengt und der Rückstand zwischen 100 ml Methylenchlorid und 50 ml Wasser verteilt. Man schüttelt die beiden Schichten und lässt sie sich dann scheiden. Die organische Schicht wird nacheinander mit Wasser und gesättigter Kochsalzlösung gewaschen und getrocknet, wobei ein Oel entsteht. Dieses wird auf einer Aluminiumoxydsäule mit Aether säulenchromatographiert, was 3-(4-Chlorphenyl)-1′-[3-(4-fluorbenzoyl)-propyl]-1,3-dihydrospiro[benzo(c)-thiophen-1,4′-piperidin]-äthylenketal als Klares Oel liefert.
Analyse:
Berechnet für $C_{30}H_{31}ClFNO_2S$: 68,76%C; 5,96%H; 2,6%N.
Gefunden: 68,63%C; 5,89%H; 2,71%N.
Das Produkt dient zur weiteren Umsetzung.

Beispiel 15

Man erhitzt ein Gemisch aus 2,0 g 3-(4-Chlorphenyl)-1,3-dihydrospiro[benzo(c)thiophen-1,4′-piperidin] Beispiel 13, 0,7 g Chlormethylcyclopropan, 1,4 g Kaliumjodid und 1,4 g Natriumbicarbonat in 20 ml Dimethylformamid 17 Stunden unter Rühren bei 80—85°C. Man lässt auf Raumtemperatur abkühlen und verdünnt dann mit 75 ml Wasser und 30 ml Methylenchlorid. Die wässrige Phase wird zweimal mit Methylenchlorid extrahiert, und sämtliche Methylenchloridlösungen werden vereinigt. Man wäscht die vereinigten Lösungen nacheinander mit Wasser und gesättigter Kochsalzlösung und trocknet dann, wobei ein klares Oel entsteht. Dieses chromatographiert man auf einer Aluminiumoxydsäule mit Aether als Eluiermittel, wobei man ein Oel erhöhter Reinheit erhält. Dieses überführt man in sein Maleinsäuresalz, das aus Aceton/Aether umkristallisiert wird, was das Salz 3-(4-Chlorphenyl)-1′-cyclopropylmethyl-1,3-dihydrospiro[benzo(c)thiophen-1,4′-piperidin]-maleinat vom Schmelzpunkt 217,5—218°C ergibt.
Analyse:
Berechnet für $C_{22}H_{24}ClNS.C_4H_4O_4$: 64,25%C; 5,81%H; 2,88%N; 7,30%Cl.
Gefunden: 64,13%C; 5,82%H; 2,77%N; 7,36%Cl.

Beispiel 16

Man rührt das Reaktionsgemisch aus 4,2 g 3-(4-Chlorphenyl)-1′-[3-(4-fluorbenzoyl)-propyl]-1,3-dihydrospiro[benzo(c)thiophen-1,4′-piperidin]-äthylenketal, dem rohen Oel aus Beispiel 14, und 22 ml 3n-Salzsäure in 60 ml Aethanol 20 Stunden bei Raumtemperatur und, nach Abkühlung auf 0°C, noch weitere 20 Stunden. Gegebenenfalls wird ein schmieriger Niederschlag durch Filtrieren entfernt und das Filtrat am Rotationsverdampfer bei 50°C eingedampft, wobei ein orangefar-bener Rückstand verbleibt. Dieser wird mit 50 ml 20%iger Natronlauge und 100 ml Aether/Methylenchlorid 1:1 ins Gleichgewicht gebracht. Man trennt die Schichten und extrahiert die wässrige Schicht mit weiteren 40 ml des besagten Aether/Methylenchloridgemischs. Die organischen Anteile werden vereinigt, mit 50 ml Aether verdünnt und dann nacheinander mit Wasser und gesättigter Kochsalzlösung gewaschen, getrocknet und unter Hinterlassung eines klaren orangefarbenen Oels eingedampft. Man chromatographiert das Oel auf einer Silikagelsäule mit 5% Methanol in Methylenchlorid als Eluiermittel, was ein gereinigtes Oel liefert, welches man in Aether auflöst und in sein Hydrobromidsalz, ein weisses Pulver, überführt. Das Pulver wird zweimal aus Methanol/Aether umkristallisiert, was das Salz 3-(4-Chlorphenyl)-1′-[3-(4-fluorbenzoyl)-propyl]-1,3-dihydrospiro[benzo(c)thiophen-1,4′-piperidin]-hydrobromid vom Schmelzpunkt 240—241°C liefert.

Analyse:
Berechnet für $C_{18}H_{22}ClFNOS.HBr$: 59,96%C; 5,03%H; 2,05%N.
Gefunden: 60,03%C; 4,85%H; 2,36%N.

Beispiel 17

a. Man rührt das Reaktionsgemisch aus 10,0 g 4-(2-Fluorphenyl)-4-hydroxy-1-methylpiperidin, Beispiel 1a, 17,5 ml 3,4-Dichlorbenzylmercaptan und 22 ml Bortrifluoridätherat in 22 ml Eisessig 16 Stunden bei 60—65°C. Danach entfernt man das Lösungsmittel bei vermindertem Druck und reibt den Rückstand mit einem Gemisch aus Aether und 1n-Salzsäure an. Man rührt zwei Studen bei 0°C und gewinnt dann den entstandenen weissen kristallinen Niederschlag durch Filtrieren. Der Niederschlag wird aus Aceton/Aether umkristallisiert, wobei farblose Nadeln, Schmelzpunkt 188—190°C, von 4-(3,4-Dichlorbenzylthio)-4-(2-fluorphenyl)-1-methylpiperidin-hydrochlorid verbleiben.

b. Man gibt 8,2 g 4-(3,4-Dichlorbenzylthio)-4-(2-fluorphenyl)-1-methylpiperidin, die freie Base aus a, in 20 ml Dimethylsulfoxyd im Verlauf von 20 Minuten vorsichtig zu einer durch 30 Minuten Erhitzen von 1,2 g 50%igem Natriumhydrid in 50 ml Dimethylsulfoxyd auf 80°C hergestellten, abgekühlten Lösung von Natriummethylsulfinylmethid. Nach beendeter Zugabe rührt man 30 Minuten bei Raumtemperatur und schreckt dann mit Wasser ab. Das zweiphasige Gemisch wird mit Methylenchlorid extrahiert und der Methylenchloridextrakt getrocknet und eingeengt, wobei ein bräunliches Oel verbleibt, das beim Stehen kristallisiert. Das Produkt wird in Aether in sein Maleinsäuresalz umgewandelt, das aus Aceton/Aether umkristallisiert wird, was das Salz 3-(3,4-Dichlorphenyl)-1,3-dihydro-1'-methylspiro[benzo(c)thiophen-1,4'-piperidin]-maleinat vom Schmelzpunkt 212—213°C ergibt.
Analyse:
Berechnet für $C_{19}H_{19}Cl_2NS.C_4H_4O_4$: 57,50%C; 4,83%H; 2,95%N; 14,76%Cl.
Gefunden: 57,75%C; 4,81%H; 2,72%N; 14,49%Cl.

Beispiel 18

Man rührt die Lösung von 2,5 g 3-(3,4-Dichlorphenyl)-1,3-dihydro-1'-methylspiro[benzo(c)thiophen-1,4'-piperidin], der freien Base aus Beispiel 17, und 1,3 g Chlorameisensäurephenylester in 30 ml Methylenchlorid 16 Stunden bei Raumtemperatur. Die gut gerührte Lösung wird nacheinander mit verdünnter Natronlauge und Wasser gewaschen und dann getrocknet, wobei ein kristallines Produkt entsteht. Dieses wird aus Aceton/Pentan umkristallisiert, was Nädelchen, Schmelzpunkt 200—202°C, von 3-(3,4-Dichlorphenyl)-1,3-dihydro-1'-phenoxycarbonylspiro[benzo(c)thiophen-1,4'-piperidin] ergibt.
Analyse:
Berechnet für $C_{25}H_{21}Cl_2NO_2S$: 63,83%C; 4,50%H; 2,98%N.
Gefunden: 63,71%C; 4,56%H; 2,93%N.
Das Produkt dient zur weiteren Umsetzung.

Beispiel 19

a. Man versetzt ein Gemisch aus 30,0 g 4-(2-Fluorphenyl)-4-hydroxy-1-methylpiperidin aus Beispiel 1a und 82 ml 2-Methylbenzylmercaptan mit 82 ml Bortrifluoridätherat. Die so erhaltene Lösung wird sechs Stunden gerührt und auf 75°C erhitzt und der Ueberschuss Reagenzien dann bei 80—100°C abdestilliert. Das im Kolben verbleibende Oel wird eine Stunde mit 250 ml 2n-HCl und 500 ml Aether verrührt und danach mit 1500 ml Eis versetzt, wobei eine breiige Masse entsteht, die man eine Stunde weiterrührt und filtriert, was einen weissen Feststoff liefert, den man mit Aether wäscht und an der Luft trocknet. Das Material wird aus Methanol/Aceton/Aether umkristallisiert, was 4-(2-Fluorphenyl)-1-methyl-4-(2-methylbenzylthio)-piperidin-hydrochlorid als Produkt ergibt. Eine Probe zur Elementaranalyse wurde nochmals aus Aceton/Aether umkristallisiert, was eine Probe mit konstantem Schmelzpunkt 206—208°C ergibt.
Analyse:
Berechnet für $C_{20}H_{24}FNS.HCl$: 65,64%C; 6,89%H; 3,83%N.
Gefunden: 65,46%C; 6,82%H; 3,55%N.

b. Eine 0.73 g-Probe Natriumhydrid, 50%ig in Mineralöl, wird durch Anreiben mit Hexan gereinigt. Man gibt 20 ml Dimethylsulfoxyd zum gereinigten Natriumhydrid und erhitzt 30 Minuten im Oelbad auf 80—90°C. Nach Abkühlen auf Raumtemperatur versetzt man im Verlauf von 5 Minuten mit 4,05 g 4-(2-Fluorphenyl)-1-methyl-4-(2-methylbenzylthio)-piperidin aus Beispiel 23a in 15 ml Dimethylsulfoxyd. Man rührt den Ansatz dreissig Minuten, giesst in 100 ml Eiswasser und filtriert. Der so erhaltene braune Feststoff wird in Dichlormethan aufgelöst und auf einer $Al_2O_3$/Aethersäule (mit Aether als Eluiermittel) chromatographiert: Schmelzpunkt 132—143°C. Die Probe wird aus Aether/Hexan umkristallisiert, wobei man 1,3-Dihydro-1'-methyl-3-(2-methylphenyl)-spiro[benzo(c)thiophen-1,4'-piperidin] vom Schmelzpunkt 132—134°C erhält.
Analyse:
Berechnet für $C_{20}H_{23}NS$: 77,62%C; 7,49%H; 4,53%N.
Gefunden: 77,63%C; 7,67%H; 4,35%N.

Beispiel 20

Man rührt eine Suspension von 3-(3,4-Dichlorphenyl)-1,3-dihydro-1'-phenoxycarbonylspiro[benzo(c)thiophen-1,4'-piperidin] (2,35 g) und 7,0 g Kaliumhydroxyd (85%) in 30 ml Aethylenglykol 30 Minuten bei 160°C. Man kühlt ab, verdünnt mit Wasser und extrahiert 3-mal mit je 100 ml Dichlormethan. Nach Trocknen ($MgSO_4$) und Eindampfen im Vakuum wandelt man das rohe Amin in Aether in ein kristallines Maleinat um. Umkristallisation aus Methanol/Aether ergibt rhombische Kristalle von 3-(3,4-Dichlorphenyl)-1,3-dihydrospiro[benzo(c)thiophen-1,4'-piperidin]-maleinat vom Schmelzpunkt 192—193°C.

Analyse:

Berechnet für $C_{18}H_{17}Cl_2NS.C_4H_4O_4$: 56,65%C; 4,54%H; 3,00%N; 15,20%Cl.

Gefunden: 56,75%C; 4,51%H; 2,88%N; 14,91%Cl.

Beispiel 21

Man rührt ein Gemisch aus 1,5 g der freien Base 3-(3,4-Dichlorophenyl)-1,3-dihydrospiro[benzo(c)thiophen-1,4'-piperidin] aus Beispiel 20, 1,3 g $\gamma$-Chlor-4-fluorbutyrophenon-äthylenketal, 0,9 g Kaliumjodid und 0,9 g Natriumbicarbonat in 15 ml wasserfreiem Dimethylformamid 16 Stunden bei 80°C. Man verdünnt mit Eiswasser und extrahiert dreimal mit $CH_2Cl_2$. Die $CH_2Cl_2$-Lösung wird getrocknet ($K_2CO_3$) und im Vakuum zu einem öligen Rückstand eingeengt. Die Reinigung erfolgt durch Säulenchromatographie (Aluminiumoxyd/Aether), und bei Behandlung mit ätherischerm HBr erhält man unter gleichzeitiger Spaltung des Ketalrings ein kristallines Hydrobromid. Umkristallisation des körnigen Hydrobromids aus Methanol/Aether ergibt 3-(3,4-Dichlorphenyl)-1'-[3-(4-fluorbenzoyl)-propyl]-1,3-dihydrospiro[benzo(c)thiophen-1,4'-piperidin]hydrobromid als farblose Kristalle.

Analyse:

Berechnet für $C_{28}H_{26}Cl_2FNOS.HBr$: 56,47%C; 4,57%H; 2,23%N.

Gefunden: 56,43%C; 4,59%H; 2,31%N.

Beispiel 22

Man rührt ein Gemisch aus der freien Base 3-(3,4-Dichlorphenyl)-1,3-dihydrospiro[benzo(c)thiophen-1,4'-piperidin] aus Beispiel 20 (2,0 g), 1,8 g $\gamma$-Chlor-4-fluorbutyrophenonäthylenketal, 0,9 g Natriumbicarbonat und 0,9 g Kaliumjodid in 15 ml wasserfreiem DMF 16 Stunden bei 80°C. Nach Abkühlung verdünnt man mit Wasser und Aether und trennt die Schichten. Die organische Lösung wird mit Wasser gewaschen, getrocknet ($MgSO_4$) und im Vakuum zu einem öligen Rückstand eingeengt. Die Reinigung des Rohprodukts gelingt, indem man es durch eine kurze Aluminiumoxyd/Aethersäule laufen lässt. Elution mit Aether ergibt ein blassgelbliches Oel, das man in Aether in ein kristallines Maleinat umwandelt. Umkristallisation aus Aceton/Aether ergibt körniges 3-(3,4-Dichlorphenyl)-1'-[3-(4-fluorbenzoyl)-propyl]-1,3-dihydrospiro[benzo(c)thiophen-1,4'-piperidin]äethylenketal-maleinat vom Schmelzpunkt 116—119°C.

Analyse:

Berechnet für $C_{30}H_{30}Cl_2FNO_2S.C_4H_4O_4$: 60,52%C; 5,08%H; 2,08%N.

Gefunden: 60,53%C; 5,05%H; 1,86%N.

Das Produkt dient zur weiteren Umsetzung.

Beispiel 23

a. Man rührt ein Gemisch aus 4-(2-Fluorphenyl)-4-hydroxy-1-methylpiperidin aus Beispiel 1a (10.5 g), 10 g p-Fluorbenzylmercaptan, 18 ml Bortrifluoridätherat und 10 ml Eisessig 16 Stunden bei 60—65°C. Der Ueberschuss Reagenzien wird unter vermindertem Druck bei 100°C entfernt und der ölige Rückstand mit überschüssiger 0,5n-Salzsäure und 200 ml Aether angerieben. Ein weisser Niederschlag wird nach 2 Stunden bei 5—10°C abfiltriert und alkalisch gemacht, was das gewünschte Produkt liefert. Das ölige Amin wird in Aether in ein kristallines Maleinat umgewandelt, das beim Umkristallisieren aus Methanol/Aether 4-(4-Fluorbenzylthio)-4-(2-fluorphenyl)-1-methylpiperidinmaleinat als farblose Prismen vom Schmelzpunkt 123—125°C ergibt.

Analyse:

Berechnet für $C_{19}H_{21}F_2NS.C_4H_4O_4$: 61,45%C; 5,61%H; 3,12%N.

Gefunden: 61,71%C; 5,75%H; 3,15%N.

b. Durch 30 Minuten Erhitzen von 0,87 g Natriumhydrid in 80 ml Dimethylsulfoxyd auf 80°C berietet man eine Lösung von Natriummethylsulfinylmethid. Dann versetzt man im Verlauf von 2 Minuten mit 8,9 g der freien Base 4-(4-Fluorbenzylthio)-4-(2-fluorphenyl)-1-methylpiperidin aus Beispiel 27a in 20 ml DMSO und rührt 15 Minuten bei 80°C weiter, bevor man mit Eiswasser abschreckt. Man extrahiert 3-mal mit $CH_2Cl_2$, wäscht die vereinigte $CH_2Cl_2$-Lösung mit Wasser, trocknet und engt zu einem öligen Rückstand ein. Reinigung des Rohprodukts erfolgt durch Säulenchromatographie (Aluminiumoxyd/Aether), und Einengen des vereinigten Ablaufs liefert ein schweres Oel, das beim Stehen kristallisiert. Umkristallisieren aus Aether/Pentan ergibt 3-(4-Fluorphenyl)-1,3-dihydro-1'-methylspiro[benzo(c)thiophen-1,4'-piperidin] als lange Nadeln vom Schmelzpunkt 109—110°C.

Analyse:
Berechnet für $C_{19}H_{20}FNS$: 72,81%C; 6,43%H; 4,47%N; 6,38%F.
Gefunden: 73,05%C: 6,31%H; 4,58%N; 6,23%F.

Beispiel 24

Man rührt ein Gemisch aus 3-(4-Fluorphenyl)-1,3-dihydro-1'-methylspiro[benzo(c)thiophen-1,4'-piperidin] aus Beispiel 23 (3,6 g) und 2,1 g Chlorameisensäurephenylester in 100 ml $CH_2Cl_2$ 64 Stunden bei Raumtemperatur. Man wäscht mit 10/iger wässriger NaOH und Wasser und trocknet über $MgSO_4$. Eindampfen bei vermindertem Druck liefert einen kristallinen Rückstand vom Schmelzpunkt 198—200°C. Beim Umkristallisieren des Rohprodukts aus Aceton/Pentan erhält man grobkristallines 3-(4-Fluorphenyl)-1,3-dihydro-1'-phenoxycarbonylspiro[benzo(c)thiophen-1,4'-piperidin] vom Schmelzpunkt 200—201°C.
Analyse:
Berechnet für $C_{25}H_{22}FNO_2S$: 71,57%C; 5,28%H; 3,34%N.
Gefunden: 71,73%C; 5,25%H; 3,20%N.
Das Produkt dient zur weiteren Umsetzung.

Beispiel 25

Man rührt ein Gemisch aus 3-(4-Fluorphenyl)-1,3-dihydro-1'-phenoxycarbonylspiro[benzo(c)thiophen-1,4'-piperidin] aus Beispiel 24 (4,3 g) und 1,4 g Kaliumhydroxyd in 50 ml Aethylenglykol 30 Minuten bei 160°C. Nach Abkühlung verdünnt man mit Wasser und extrahiert dreimal mit je 150 ml Aether. Der vereinigte Aetherextrakt wird mit Wasser gewaschen (3- bis 4-mal), getrocknet und zu einem öligen Rückstand eines sekundären Amins eingeengt. Dieses wird in herkömmlicher Weise durch mehrstündiges Rühren mit Salzsäure ohne Erhitzen in sein Hydrochlorid umgewandelt. Umkristallisation aus Methanol/Aceton/Aether ergibt 3-(4-Fluorphenyl)-1,3-dihydrospiro[benzo(c)thiophen-1,4'-piperidin]-hydrochlorid als Kristalle vom Schmelzpunkt 259—260°C.
Analyse:
Berechnet für $C_{18}H_{18}FNS.HCl$: 64,36%C; 5,70%H; 4,17%N; 5,65%F.
Gefunden: 64,09%C; 5,55%H; 4,28%N; 5,78%F.

Beispiel 26

Man rührt ein Gemisch aus 3-(4-Fluorphenyl)-1,3-dihydrospiro[benzo(c)thiophen-1,4'-piperidin] (2,2 g der freien Base aus Beispiel 25), 2,2 g $\gamma$-Chlor-4-fluorbutyrophenonäthylenketal, 1,5 g Natriumbicarbonat und 1,5 g Kaliumjodid in 25 ml DMF 16 Stunden bei 80°C. Nach Abkühlung schreckt man mit Wasser ab, extrahiert 3-mal mit Aether und trocknet. Beim Entfernen des Lösungsmittels im Vakuum bei 80°C verbleibt ein rötliches Oel, welches, gereinigt wird, indem man es durch eine kurze, mit Aluminiumoxyd in Aether gefüllte Säule laufen lässt. Elution mit Aether ergibt ein reines tertiäres Amin, das in ein kristallines Maleinat umgewandelt wird. Umkristallisation aus Aceton/Aether liefert 1'-[3 - (4 - Fluorbenzoyl) - propyl] - 3 - (4 - fluorphenyl) - 1,3 - dihydrospiro[benzo(c)thiophen - 1,4' - piperidin] - äthylenketalmaleinat als farblose Prismen vom Schmelzpunkt 175—177°C.
Analyse:
Berechnet für $C_{30}H_{31}F_2NO_2S.C_4H_4O_4$: 65,46%C; 5,64%H; 2,24%N.
Gefunden: 65,28%C; 5,71%H; 2,21%N.
Das Produkt dient zur weiteren Umsetzung.

Beispiel 27

Man sättigt die Lösung von 1'-[3-(p-Fluorbenzoyl)-propyl]-3-(4-fluorphenyl)-1,3-dihydrospiro[benzo(c)thiophen-1,4'-piperidin]-äthylenketal aus Beispiel 26 (2,3 g der freien Base) in 200 ml Aether und 10 ml Methanol mit wasserfreiem Bromwasserstoff. Nach 2 Stunden Stehen be Raumtemperatur neutralisiert man vorsichtig mit 1n-Ammoniak, wäscht die Aetherlösung 3-mal mit Wasser, trocknet und engt zu einem öligen Rückstand ein. Die freie Base wird in Aether in ein kristallines Maleinat umgewandelt, und Umkristallisation aus Aceton/Aether ergibt 1'-[3-(4-Fluorbenzoyl)-propyl]-3-(4-fluorphenyl)-1,3-dihydrospiro[benzo(c)thiophen-1,4'-piperidin]-maleinat als weissliche Prismen vom Schmelzpunkt 149—150°C.
Analyse:
Berechnet für $C_{28}H_{27}F_2NOS.C_4H_4O_4$: 66,31%C; 5,40%H; 2,42%N; 6,55%F.
Gefunden: 66,41%C; 5,22%H; 2,36%N; 6,64%F.

Beispiel 28

a. Man versetzt 25 g 4-(2-Fluorphenyl)-4-hydroxy-1-methylpiperidin aus Beispiel 1a in 55 ml Essigsäure mit 59,7 g 2-Chlorbenzylmercaptan und danach mit 55 ml Bortrifluoridätherat. Man rührt den Ansatz 20 Stunden bei 55—65°C. Ueberschüssige Säure wird unter vermindertem Druck bei 80—100°C entfernt. Den öligen Rückstand bringt man dann mit 200 ml 2n-Salzsäure und 200 ml Aether ins Gleichgewicht. De Aether sowie zwei weitere 125 ml-Portionen Aether werden abgegossen. Das Oel/Wassergemisch wird mit Wasser auf ein Volumen von 650 ml verdünnt und etwa drei

Tage bei 0°C gelagert, um Kristalle sich bilden zu lassen. Das Gemisch wird verdünnt und filtriert. Der Niederschlag wird mit Wasser und Aether gewaschen, getrocknet und zu einem unscharf schmelzenden (122—129°C) Feststoff umkristallisiert (Aceton/Aether). Ein Teil des Feststoffs wird in Wasser gegeben, mit 10%iger wässriger NaOH alkalisch gemacht, in Aether extrahiert, gewaschen, getrocknet und mit ätherischem Chlorwasserstoff behandelt, was ein weisses Pulver ergibt. Dieses wird aus Aceton/Aether umkristallisiert, wobei man 4-(2-Chlorbenzylthio)-4-(2-fluorphenyl)-1-methylpiperidin-hydrochlorid als weissen kristallinen Feststoff vom Schmelzpunkt 173—175°C erhält.

Analyse:
Berechnet für $C_{19}H_{21}ClFNS$: 59,08%C; 5,74%H; 3,63%N; 4,92%F.
Gefunden: 58,81%C; 5,63%H; 3,37%N; 5,03%F.

b. Eine 0,2 g-Probe Natriumhydrid (durch Waschen einer 50%igen Oeldispersion bereitet) wird in 15 ml Dimethylsulfoxyd unter Rühren 30 Minuten auf 80°C erhitzt. Dann versetzt man auf einmal mit 2,1 g der freien Base 4-(2-Chlorbenzylthio)-4-(2-fluorphenyl)-1-methylpiperidin aus Beispiel 28a in 10 ml trockenem DMSO und entfernt das Oelbad. Man lässt ohne Erhitzen 40 Minuten weiterrühren und giesst dann auf 250 cm³ Eis mit 250 ml Aether. Die Schichten werden umgeschüttelt und getrennt und der wässrige Anteil nochmals mit 150 ml Aether extrahiert. Den vereinigten Aether wascht man zweimal mit je 100 ml Wasser und einmal mit 10 ml gesättigter Kochsalzlösung und trocknet über Magnesiumsulfat, was ein Oel ergibt. Dieses wird auf einer Aluminiumoxyd/Aethersäule zu einem Material (freie Base) chromatographiert, das bei Dünnschichtchromatographie nur einen Fleck (Rf 0,54; 25% $MeOH/CH_2Cl_2$, Siliciumdioxyd) zeigt. Das Produkt wird in Aether mit überschüssiger ätherischer Maleinsäure behandelt, mit Aether gewaschen und getrocknet, was 3-(2-Chlorphenyl)-1,3-dihydro-1'-methylspiro[benzo(c)thiophen-1,4'-piperidin]-maleinat als weisses Pulver vom Schmelzpunkt 173—176°C ergibt.

Ein kleiner Anteil des Salzes wird zweimal aus Aceton umkristallisiert, wobei man bei 178—178,5°C schmelzende Kristalle erhält.

Analyse:
Berechnet für $C_{19}H_{20}ClNS.C_4H_4O_4$: 61,95%C; 5,43%H; 3,14%N; 7,95%Cl.
Gefunden: 62,03%C; 5,46%N; 3,10%N; 7,94%Cl.

## Beispiel 29

a. Man rührt ein Gemisch aus 4-(2-Fluorphenyl)-4-hydroxy-1-methylpiperidin aus Beispiel 1a (15,8 g), 12 ml 2-Fluorbenzylmercaptan und 20 ml Bortrifluoridätherat in 15 ml Eisessig 16 Stunden bei 65—70°C. Der Ueberschuss Reagenzien wird bei vermindertem Druck entfernt und der Rückstand mit 300 ml 0,5n-HCl und 100 ml Aether angerieben. Nach 30 Minuten Stehen bei 5—10°C filtriert man den kristallinen Niederschlag ab, trocknet an der Luft und macht mit 10%igem wässrigen $NH_4OH$ alkalisch. Das freigesetzte Amin wird in Aether aufgenommen, getrocknet und zu einem gelblichen Oel eingeengt, das man dann in Aether in sein Maleinat umwandelt; Umkristallisation des rohen Salzes aus Methanol/Aether ergibt 4-(2-Fluorbenzylthio)-4-(2-fluorphenyl)-1-methylpiperidin-maleinat als farblose Prismen vom Schmelzpunkt 153—154°C.

Analyse:
Berechnet für $C_{19}H_{21}F_2NS.C_4H_4O_4$: 61,45%C; 5,61%H; 3,12%N.
Gefunden: 61,28%C; 5,57%H; 2,98%N.

b. Durch 30 Minuten Erhitzen von 1,2 g Natriumhydrid in 100 ml DMSO bei 80°C bereitet man eine Lösung von Natriummethylsulfinylmethid. Man kühlt auf Raumtemperatur ab und versetzt im Verlauf von 5 Minuten mit 12 g der freien Base 4-(2-Fluorbenzylthio)-4-(2-fluorphenyl)-1-methylpiperidin in 40 ml DMSO. Nach der Zugabe rührt man noch 30 Minuten bei 70—80°C weiter. Man verdünnt mit Eiswasser und extrahiert 3-mal mit Aether. Die vereinigte Aetherlösung wird 4-mal mit Wasser gewaschen, getrocknet und zu einem dicken Oel eingeengt. Chromatographie über $Al_2O_3$/Aether entfernt eine geringe Menge Verunreinigungen, und das gereinigte Amin wird in Aether in ein kristallines Hydrobromid umgewandelt. Umkristallisation aus Methanol/Aether ergibt 3-(2-Fluorphenyl)-1,3-dihydro-1'-methylspiro-[benzo(c)thiophen-1,4'-piperidin]-hydrobromid als farblose Kristalle vom Schmelzpunkt 263—265°C (Zersetzung).

Analyse:
Berechnet für $C_{19}H_{20}FNS.HBr$: 57,86%C; 5,37%H; 3,55%N; 4,82%F.
Gefunden: 57,67%C; 5,47%H; 3,58%N; 4,80%F.

## Beispiel 30

Man rührt ein Gemisch aus dem freien Amin 3-(2-Fluorphenyl)-1,3-dihydro-1'-methylspiro[benzo(c)thiophen-1,4'-piperidin] aus Beispiel 29 (6,3 g) und 3,9 g Chlorameisensäurephenylester in 100 ml wasserfreiem $CH_2Cl_2$ 4 Stunden bei Raumtemperatur. Man wäscht das Reaktionsgemisch mit 10%iger wässriger Natronlauge und trocknet 2 Stunden über $MgSO_4$.

Entfernung des Lösungsmittels bei vermindertem Druck führt zu einem dicken Oel, welches man durch eine kurze, mit Silikagel in $CH_2Cl_2$ gefüllte Säule reinigt. Elution mit einem grossen Ueberschuss $CH_2Cl_2$ ergibt nach dem Einengen ein farbloses Oel, welches beim Stehen erstarrt und 3-(2-Fluorphenyl)-1,3-dihydro-1′-phenoxycarbonylspiro[benzo(c)thiophen-1,4′-piperidin] vom Schmelzpunkt 122—123°C liefert.

Analyse:

Berechnet für $C_{25}H_{22}FNO_2S$: 71,57%C; 5,28%H; 3,34%N.

Gefunden: 71,49%C; 5,23%H; 3,27%N.

Das Produkt dient zur weiteren Umsetzung.

Beispiel 31

Man rührt ein Gemisch aus 3-(2-Fluorphenyl)-1,3-dihydro-1′-phenoxycarbonylspiro[benzo(c)thiophen-1,4′-piperidin] aus Beispiel 30 (5,6 g) und 18 g Kaliumhydroxyd in 100 ml Aethylenglykol 30 Minuten bei 170°C. Man giesst in Wasser und extrahiert 3-mal mit Aether. Die Aetherlösung wird mit Wasser gewaschen, getrocknet und im Vakuum zu einem viskosen Oel von freiem Amin eingeengt. Man stellt ein kristallines Maleinat her und kristallisiert aus Methanol/Aceton/Aether um, was 3-(2-Fluorphenyl)-1,3-dihydrospiro[benzo(c)thiophen-1,4′-piperidin]-maleinat als weisses Pulver ergibt.

Analyse:

Berechnet für $C_{18}H_{18}FNS.C_4H_4O_4$: 63,59%C; 5,34%H; 3,37%N; 4,57%F.

Gefunden: 63,55%C; 5,33%H; 3,10%N; 4,52%F.

Beispiel 32

Man rührt ein Gemisch aus 3-(2-Fluorphenyl)-1,5-dihydrospiro[benzo(c)thiophen-1,4′-piperidin] aus Beispiel 31 (2,2 g), 2,2 g der Aethylenketalverbindung von $\gamma$-Chlor-4-fluorbutyrophenon, 1,5 g Kaliumjodid und 1,5 g Natriumbicarbonat in 25 ml DMS 16 Stunden bei 70—75°C. Nach Abkühlung verdünnt man mit Wasser und extrahiert 3-mal mit Aether. Nach Waschen mit Wasser und Trocknen engt man die Aetherlösung im Vakuum zu einem öligen Rückstand ein und reinigt diesen durch eine kurze Aluminiumoxydsäule, wobei Elution mit Aether ein farbloses Oel ergibt, welches man in ein kristallines Maleinat, Schmelzpunkt 150—151°C, von 1′-[3-(4-Fluorbenzoyl)-propyl]-3-(2-fluorphenyl)-1,3-dihydrospiro[benzo(c)thiophen-1,4′-piperidin]-äthylenketal überführt.

Analyse:

Berechnet für $C_{30}H_{30}F_2NO_2S.C_4H_4O_4$: 65,46%C; 5,64%H; 2,24%N.

Gefunden: 65,46%C; 5,37%H; 2,12%N.

Das Produkt dient zur weiteren Umsetzung.

Beispiel 33

Man erhitzt die Lösung von 1′-[3-(4-Fluorbenzoyl)-propyl]-3-(2-fluorphenyl)-1,3-dihydrospiro[benzo(c)thiophen-1,4′-piperidin]-äthylenketal aus Beispiel 32 (2,0 g) in 20 ml Aethanol und 20 ml 3n-Salzsäure 30 Minuten auf dem Dampfbad. Nach Abkühlung macht man mit 40%iger wässriger Natronlauge alkalisch und extrahiert 3-mal mit Aether. Die vereinigte Aetherlösung wird gründlich mit Wasser gewaschen, getrocknet und mit überschüssiger ätherischer Maleinsäure behandelt. Das kristalline Maleinat wird aus Aceton/Aether umkristallisiert, wobei man 1′-[3-(4-Fluorbenzoyl)-propyl]-3-(2-fluorphenyl)-1,3-dihydrospiro[benzo(c)thiophen-1,4′-piperidin]-maleinat als völlig weisse Prismen vom Schmelzpunkt 137—138,5°C erhält.

Analyse:

Berechnet für $C_{28}H_{27}F_2NO.C_4H_4O_4$: 66,31%C; 5,40%H; 2,42%N; 6,55%F.

Gefunden: 66,18%C; 5,34%H; 2,42%N; 6,49%F.

Beispiel 34

Man rührt eine 4,5 g-Probe Chlorameisensäurephenylester in 150 ml Dichlormethan bei 25°C, während man 7,5 g 1,3-Dihydro-1′-methyl-3-(2-methylphenyl)-spiro[benzo(c)thiophen-1,4′-piperidin] aus Beispiel 19 in 75 ml Dichlormethan rasch dazugibt. Der Ansatz wird insgesamt 24 Stunden bei 25°C gerührt und mit 225 ml 10%iger wässriger NaOH-lauge abgeschreckt. Die organische Schicht wird abgetrennt, mit Wasser gewaschen, über $MgSO_4$ getrocknet, filtriert und zu einem Oel eingedampft. Das Oel säulenchromatographiert man auf einer Silikagel/$CH_2Cl_2$-säule unter Verwendung von $CH_2Cl_2$ zur Elution. Als Produkt isoliert man 1,3-Dihydro-3-(2-methylphenyl)-1′-phenoxycarbonylspiro[benzo(c)thiophen-1,4′-piperidin] als schwachgelbes Oel.

Analyse:

Berechnet für $C_{26}H_{25}NO_2S$: 75,15%C; 6.06%H.

Gefunden: 74,90%C; 6.05%H.

Das Produkt dient zur weiteren Umsetzung.

Beispiel 35

Man gibt die Lösung von 16 g in 100 ml Aethylenglykol gelöstem Kaliumhydroxyd zu 6,0 g 1,3-Dihydro-3-(2-methylphenyl)-1′-phenoxycarbonylspiro[benzo(c)thiophen-1,4′-piperidin] aus Beispiel 34. Die so erhaltene Lösung wird eine Stunde gerührt und auf 160°C erhitzt. Man giesst in 300 ml Eiswasser und extrahiert mit Aether. Die Aetherfraktionen werden vereinigt, mit Wasser gewaschen, über

$MgSO_4$ getrocknet, filtriert und zu einem Rückstand eingedampft, den man in ein weisses, körniges Maleinatsalz, 1,3-Dihydro-3-(2-methylphenyl)-spiro[benzo(c)thiophen-1,4'-piperidin]-maleinat, umwandelt, das aus Methanol/Aether umkristallisiert wird und einen Schmelzpunkt von 178—179°C besitzt.

Analyse:
Berechnet für $C_{19}H_{21}NS.C_4H_4O_4$: 67,13%C; 6,12%H; 3,40%N.
Gefunden: 67,10%C; 5,96%H; 3,14%N.

Beispiel 36

Ein Gemisch aus 6,0 g 1,3-Dihydro-3-(4-methylphenyl)-1'-phenoxycarbonylspiro[benzo(c)thiophen-1,4'-piperidin] aus Beispiel 11, 16,0 g Kaliumhydroxyd und 100 ml Aethylenglykol wird eine Stunde gerührt und bei 160°C erhitzt. Man schreckt mit Eiswasser ab und extrahiert mit Aether. Die Aetherextrakte werden vereinigt, mit Wasser gewaschen, über $MgSO_4$ getrocknet und zu einem Oel eingedampft. Das Oel wird in 1,3-Dihydro-3-(4-methylphenyl)-spiro[benzo(c)thiophen-1,4'-piperidin]-maleinat überführt. Schmelzpunkt 214—215°C.

Analyse:
Berechnet für $C_{19}H_{21}NS.C_4H_4O_4$: 67,13%C; 6,12%H; 3,40%N.
Gefunden: 67,05%C; 6,20%H; 3,21%N.

Beispiel 37

a. Man versetzt 25 g 4-(2-Fluorphenyl)-4-hydroxy-1-methylpiperidin aus Beispiel 1a mit 50 g m-Methylbenzylmercaptan. Dazu gibt man 68 ml Bortrifluoridätherat. Man rührt etwa drei Tage bei 65—70°C. Ueberschüssiges Reagenz wird dann unter Saugflaschendruck bei 60—100°C entfernt. Man giesst in 360 ml 0,5n-Salzsäure mit 300 ml Aether. Wasser und Aether werden von dem ausfallenden Oel abgegossen. Das Oel wird unter 250 ml 0,5n-Salzsäure und 200 ml Aether eingetragen. Man trennt das Oel wiederum von den Lösüngen und trägt es unter 150 ml Wasser mit 20 ml 10%iger wässriger Natronlauge ein. Die Suspension wird mit 200 ml Wasser weiter verdünnt und mit zweimal je 200 ml und einmal mit 100 ml Aether extrahiert. Die vereinigten Aminätherextrakte werden zu einer Lösung von 12,5 ml 48%igem wässrigen Bromwasserstoff in 300 ml Wasser gegeben, was ein weisses Salz liefert. Man lässt 16 Standen stehen und giesst dann den Aether ab. Das wässrige Gemisch wird nochmals durch Zugabe und Abgiessen von 200 ml Aether gewaschen. Das Salz wird dann abfiltriert, mit Wasser und Aether gewaschen und zu einem weissen Pulver, Schmelzpunkt 143—145°C, von 4-(2-Fluorphenyl)-4-(3-methylbenzylthio)-1-methylpiperidinhydrobromid getrocknet. Eine dreimal umkristallisierte (Aceton/Aether) Probe schmilzt bei 145—146°C.

Analyse:
Berechnet für $C_{20}H_{24}FNS.HBr$: 58,54%C; 6,14%H; 3,4%N.
Gefunden: 58,45%C; 6,16%H; 3,16%N.

b. Eine 3,43 g-Portion 50%ige Natriumhydriddispersion wird unter trockenem Stickstoff mit Hexan gewaschen und dann 40 Minuten mit 100 ml siebgetrocknetem Dimethylsulfoxyd auf 80—85°C erhitzt. Dann versetzt man im Verlauf von 60 Sekunden mit 18,48 g der freien Base 4-(2-Fluorphenyl)-4-(3-methylbenzylthio)-1-methylpiperidin aus Beispiel 37a in 70 ml trockenem DMSO. Das rote Gemisch wird gerührt, im Verlauf einer Stunde auf Raumtemperatur abgekühlt und in 500 $cm^3$ Eiswasser gegossen. Man extrahiert das wässrige Gemisch dreimal mit je 200 ml Dichlormethan. Die vereinigten Dichlormethanextrakte werden viermal mit je 200 ml Wasser und einmal mit 100 ml gesättigter wässriger Kochsalzlösung gewaschen und über Magnesiumsulfat getrocknet, was ein Oel liefert. Dieses löst man in Aether, filtriert, dampft ein und chromatographiert mit Aether über 400 ml Aluminiumoxyd, was ein Oel ergibt, welches man in Aether auflöst und zwecks Salzbildung mit ätherischem Bromwasserstoff behandelt. Das Salz wird mit Aether gewaschen und aus Methanol/Aceton/Aether umkristallisiert, was 1,3-Dihydro-1'-methyl-3-(3-methylphenyl)-spiro[benzo(c)thiophen-1,4'-piperidin]-hydrobromid als weisses kristallines Pulver vom Schmelzpunkt 258—260°C ergibt.

Analyse:
Berechnet für $C_{20}H_{23}NS.HBr$: 61,54%C; 6,20%H; 3,59%N.
Gefunden: 61,27%C; 6,24%H; 3,45%N.

Beispiel 38

Die Lösung von 0,70 g des Amins 1,3-Dihydro-3-(2-methylphenyl)-spiro[benzo(c)thiophen-1,4'-piperidin] aus Beispiel 35, 0,76 g $\gamma$-Chlor-p-fluorbutyrophenon-äthylenketal, 0,38 g Natriumbicarbonat, 0,38 g KJ und 10 ml wasserfreiem DMF wird 24 Stunden gerührt und bei 70—80°C erhitzt. Der Ansatz wird durch Eingiessen in Wasser und Extraktion mit Aether aufgearbeitet. Die Aetherfraktionen werden vereinigt, mit Wasser gewaschen und über $MgSO_4$ getrocknet. Man filtriert die Lösung, dampft ein und säulenchromatographiert das erhaltene Oel auf einer $Al_2O_3$/Aethersäule (mit Aether eluiert). Das isolierte Produkt wird in ein Maleinatsalz, Schmelzpunkt 157—159°C, von 1'-[3-(4-Fluorbenzoyl)propyl] - 1,3 - dihydro - 3 - (2 - methylphenyl) - spiro[benzo(c)thiophen - 1,4' - piperidin] - äthylenketal überführt.

Analyse:
Berechnet für $C_{31}H_{34}FNO_2S.C_4H_4O_4$: 67,83%C; 6,18%H; 2,26%N.
Gefunden: 67,78%C; 6,02%H; 2,24%N.
Das Produkt dient zur weiteren Umsetzung.

Beispiel 39

Man versetzt 3,83 g Chlorameisensäurephenylester in 850 ml Dichlormethan im Verlauf von zwei Minuten mit der Lösung von 6,73 g der freien Base 3-(2-Chlorphenyl)-1,3-dihydro-1′-methyl-spiro[benzo(c)thiophen-1,4′-piperidin] aus Beispiel 28 in 75 ml Dichlormethan. Man rührt 64 Stunden bei Raumtemperatur, verdünnt dann mit 200 ml Dichloromethan und wäscht mit zweimal je 300 ml 5%iger wässriger Natronlauge, zweimal je 200 ml Wasser und einmal 75 ml Salzlösung. Man trocknet über Magnesiumsulfat und dampft zu einem Oel ein. Dieses chromatographiert man über Silikagel mit Dichlormethan, was 3-(2-Chlorphenyl)-1,3-dihydro-1′-phenoxycarbonylspiro[benzo(c)thiophen-1,4′-piperidin] als Oel liefert.
Analyse:
Berechnet für $C_{25}H_{22}ClNO_2S$: 68,88%C; 5,09%H; 3,21%N.
Gefunden: 69,36%C; 4,94%H: 3,33%N.
Das Produkt dient zur weiteren Umsetzung.

Beispiel 40

Man versetzt 1,81 g Chlorameisensäurephenylester in 40 ml Dichlormethan im Verlauf von 5 Minuten mit der Lösung von 2,98 g der freien Base 1,3-Dihydro-1′-methyl-3-(3-methylphenyl)-spiro[benzo(c)thiophen-1,4′-piperidin] aus Beispiel 37 in 35 ml Dichlormethan. Man rührt 21 Stunden bei Raumtemperatur. Dann wird der Ansatz mit 100 ml Dichlormethan verdünnt, zweimal mit je 150 ml 5%iger wässriger Natronlauge, zweimal mit je 100 ml Wasser und einmal mit 50 ml Salzlösung gewaschen und über Magnesiumsulfat zu einem Oel getrocknet. Dieses chromatographiert man über Silikagel mit Dichlormethan, was 1,3-Dihydro-3-(3-methylphenyl)-1′-phenoxycarbonylspiro[benzo(c)thiophen-1,4′-piperidin], Schmelzpunkt 154—157°C, liefert. Ein Teil davon wird aus Toluol/Hexan umkristallisiert, Schmelzpunkt 157—160°C.
Analyse:
Berechnet für $C_{26}H_{25}NO_2S$: 75,16%C; 6,07%H; 3,37%N.
Gefunden: 75,28%C; 6,12%H; 3,17%N.
Das Produkt dient zur weiteren Umsetzung.

Beispiel 41

Man gibt 15,1 g 85%ige Kaliumhydroxyplätzchen bei 150°C zu 6,19 g 3-(2-Chlorphenyl)-1,3-dihydro-1′-phenoxycarbonylspiro[benzo(c)thiophen-1,4′-piperidin] aus Beispiel 39 in 95 ml Aethylenglykol. Der Ansatz wird in einem Bad von 160—165°C 40 Minuten gerührt, dann auf Raumtemperatur abgekühlt und in 250 ml Wasser gegossen. Man verdünnt mit Wasser auf 500 ml und extrahiert mit dreimal je 150 ml Aether, wäscht mit dreimal je 150 ml Wasser und einmal mit 40 ml gesättigter Kochsalzlösung und trocknet über Magnesiumsulfat zu einem Oel. Dieses löst man in Aether, filtriert und behandelt mit ätherischer Maleinsäure. Das rohe Salz wird mit Aether gewaschen und aus Methanol/Aceton/Aether umkristallisiert, was 3-(2-Chlorphenyl)-1,3-dihydrospiro[benzo(c)thiophen-1,4′-piperidin]-maleinat als weisses Salz vom Schmelzpunkt 172—173°C ergibt.
Analyse:
Berechnet für $C_{18}H_{18}ClNS.C_4H_4O_4$: 61,18%C; 5,14%H; 3,24%N; 8,21%Cl.
Gefunden: 61,47%C; 5,22%H; 3,18%N; 7,85%Cl.

Beispiel 42

Man gibt 2,02 g Natriumbicarbonat und 2,02 g Kaliumjodid zu 2,48 g der freien Base 3-(2-Chlorphenyl)-1,3-dihydro-spiro[benzo(c)thiophen-1,4′-piperidin] aus Beispiel 41 in 28 ml seibgetrocknetem Dimethylformamid mit 2,78 g $\gamma$-Chlor-p-fluorphenylbutyrophenon-äthylenketal. Dann erhitzt man den Ansatz 17 Stunden in einem 85—90°C heissen Bad. Man verdünnt mit 90 ml Chloroform und filtriert durch Papier. Die lösung wird eingedampft und das Oel zwischen 140 ml Dichlormethan mit 75 ml Wasser verteilt. Man wäscht das Dichlormethan mit 75 ml Wasser und einmal 25 ml gesättigter Kochsalzlösung und trocknet über Magnesiumsulfat. Das Oel wird über Aluminiumoxyd mit Aether chromatographiert und zur Salzbildung mit ätherischer Oxalsäure behandelt. Das Salz wird mit Aether gewaschen und aus Methanol/Aceton/Aether umkristallisiert, was 3-(2-Chlorphenyl)-1′-[3-(4-fluorbenzoyl)-propyl]-1,3-dihydrospiro[benzo(c)thiophen-1,4′-piperidin]-äthylenketal-oxalat als weissen Feststoff vom Schmelzpunkt 200—201°C ergibt.
Analyse:
Berechnet für $C_{30}H_{31}ClFNO_2S.C_2H_2O_4$: 62,59%C; 5,42%H; 2,28%N.
Gefunden: 62,53%C; 5,44%H; 2,26%N.
Das Produkt dient zur weiteren Umsetzung.

Beispiel 43

a. Man versetzt 14,45 g 4-(2-Fluorphenyl)-4-hydroxy-1-methylpiperidin aus Beispiel 1a mit 39,45 g 2,4-Dichlorbenzylmercaptan und danach mit 40 ml Bortrifluoridätherat. Man rührt etwa 4 Tage bei 80—85°C. Ueberschüssiges Reagenz wird bei Saugflaschendruck bis 110°C entfernt und das verbleibende Oel in 200 ml 0,5n-Salzsäure und 200 ml Aether gegossen. Man lässt 18 Stunden stehen, giesst den Aether ab, gibt noch zweimal je 200 ml Aether dazu und giesst ab. Der rohe Niederschlag wird abfiltriert, mit 75 ml Wasser und dreimal je 125 ml Aether gewaschen und zu einem anscheinend gemischten Salz getrocknet. Ein Teil des Salzes wird mit wässrigem Ammoniumhydroxyd alkalisch gemacht, mit Aether extrahiert, mit Wasser und Salzlösung gewaschen und über Magnesiumsulfat getrocknet. Die ätherische Lösung behandelt man zur Salzbildung mit ätherischem Bromwasserstoff. Das Salz wird mit Aether gewaschen und getrocknet, was 4-(2,4-Dichlorbenzylthio)-4-(2-fluorphenyl)-1-methylpiperidinhydrobromid als weisses Pulver vom Schmelzpunkt 208,5—210°C ergibt.

Analyse:
Berechnet für $C_{19}H_{20}Cl_2FNS.HBr$: 49,05%C; 4,55%H; 3,01%N.
Gefunden: 48,87%C; 4,52%H; 2,96%N.

b. Man wäscht 0,3 g Natriumhydrid (50%) unter Stickstoff mit Hexan und erhitzt 30 Minuten mit 11 ml trockenem Dimethylsulfoxyd auf 80°C. Dann versetzt man auf einmal mit 1,68 g der freien Base 4-(2,4-Dichlorbenzylthio)-4-(2-fluorphenyl)-1-methylpiperidin aus Beispiel 43a in 7,3 ml trockenem DMSO und entfernt die Heizung. Während des Abkühlens auf Raumtemperatur im Verlauf von 45 Minuten wird der Ansatz unter Stickstoff gerührt, dann auf Eis gegossen und mit 50 ml Aether und 150 ml Dichlormethan extrahiert. Die vereinigten organischen Phasen wäscht man mit zweimal je 100 ml Wasser und einmal 25 ml Salzlösung und trocknet über Magnesiumsulfat zu einem Oel. Dieses wird über Aluminiumoxyd mit Aether chromatographiert, was ein Oel ergibt, welches man zur Salzbildung in Aether mit ätherischem Bromwasserstoff behandelt. Das Salz wird mit Aether gewaschen und aus Aceton/Aether umkristallisiert, was 3-(2,4-Dichlorphenyl)-1,3-dihydro-1′-methylspiro[benzo(c)thiophen-1,4′-piperidin]-hydrobromid als gelblichweisses Salz vom Schmelzpunkt 259,5—261°C ergibt. Ein Teil des rohen Salzes wird dreimal aus Methanol/Aceton/Aether umkristallisiert, wonach der Schmelzpunkt 260—261°C beträgt.

Analyse:
Berechnet für $C_{19}H_{19}Cl_2NS.HBr$: 51,26%C; 4,53%H; 3,15%N; 15,93%Cl.
Gefunden: 51,43%C; 4,56%H; 3,15%N; 15,65%Cl.

Beispiel 44

Man versetzt 1,34 g 1,3-Dihydro-3-(3-methylphenyl)-1′-phenoxycarbonylspiro[benzo(c)thiophen-1,4′-piperidin] aus Beispiel 40 in 22 ml Aethylenglykol unter Stickstoff bei 150°C mit 3,44 g 85%iger wässriger Kalilauge. Man rührt 40 Minuten bei 160°C, giesst dann in 50 ml Eiswasser, verdünnt auf 120 ml und extrahiert mit zweimal je 50 ml Dichlormethan. Der vereinigte Dichlormethanextrakt wird mit zweimal je 50 ml Wasser und einmal mit 20 ml gesättigter Kochsalzlösung gewaschen und über Magnesiumsulfat zu einem Oel getrocknet. Durch Umschwenken mit einmal 50 ml und zweimal je 25 ml siedendem Hexan und jeweils Abgiessen wird das Oel gereinigt. Nach Eindampfen des Hexans ergibt sich ein Oel (freie Base), das man in Aether löst, mit ätherischer Maleinsäure behandelt und aus Aceton zu einem weissen Pulver, Schmelzpunkt 175—176°C, von 1,3-Dihydro-3-(3-methylphenyl)-spiro[benzo(c)thiophen-1,4′-piperidin]-maleinat umkristallisiert.

Analyse:
Berechnet für $C_{19}H_{21}SN.C_4H_4O_4$: 67,14%C; 6,12%H; 3,41%N.
Gefunden: 67,29%C; 6,13%H; 3,30%N.

Beispiel 45

Man versetzt 4,02 g der freien Base 3-(2-Chlorphenyl)-1′-[3-(4-fluorbenzoyl)-propyl]-1,3-dihydrospiro[benzo(c)thiophen-1,4′-piperidin]-äthylenketal aus Beispiel 42 in 250 ml Aether sowie 15 ml Methanol mit 120 ml ätherischer Bromwasserstofflösung. Man lässt zwei Stunden rühren, und gibt dann unter Rühren 120 ml 58%iges Ammoniumhydroxyd/Wasser (1:4) dazu, um alles aufzulösen. Die Schichten werden geschieden und der Aether dreimal mit je 100 ml Wasser und einmal mit 25 ml gesättigter wässriger Kochsalzlösung gewaschen und über Magnesiumsulfat getrocknet, was einen Feststoff liefert. Dieser wird eine Stunde in einem Gemisch aus 125 ml Aether und 64 ml Wasser/58%iges Ammoniumhydroxyd (15:1) verrührt. Die Schichten werden geschieden und die wässrige Schicht mit zweimal je 50 ml Aether gewaschen, der dann filtriert und getrocknet wird. Die vereinigten Aetherlösungen wäscht man mit Wasser und Salzlösung, trocknet, dampft ein und kristallisiert die Feststoffe aus Essigester um, was zusammen 3-(2-Chlorphenyl)-1′-[3-(4-fluorbenzoylpropyl]-dihydrospiro[benzo(c)thiophen-1,4′-piperidin] als bei 144—148°C schmelzenden Feststoff liefert.

Analyse:
Berechnet für $C_{28}H_{27}ClFNOS$: 70,07%C; 5,67%H; 2,92%N; 7,39%Cl.
Gefunden: 69,81%C; 5,54%H; 2,79%N; 7,29%Cl.

Beispiel 46

Man versetzt 5,25 g der freien Base 1,3-Dihydro-3-(3-methylphenyl)-spiro[benzo(c)thiophen-1,4'-piperidin] aus Beispiel 44 in 65 ml siebgetrocknetem Dimethylformamid mit 5,43 g $\gamma$-Chlor-p-fluorbutyrophenon-äthylenketal und danach mit 3,6 g Natriumbicarbonat und 3,6 g Kaliumjodid. Man rührt 24 Stunden in einem 85°C heissen Bad, verdünnt dann mit 130 ml Chloroform und filtriert. Das Lösungsmittel wird entfernt und das zurückbleibende Oel zwischen 250 ml Dichlormethan und einmal 150 ml Wasser sowie einmal 35 ml gesättigter Kochsalzlösung verteilt und über Magnesiumsulfat zu einem Oel getrocknet. Dieses chromatographiert man über Aluminiumoxyd mit Aether und überführt es durch Behandlung mit ätherischer Maleinsäure in das Maleinatsalz. Umkristallisation aus Aceton/Aether liefert 1'-[3-(4-Fluorbenzoyl)-propyl]-1,3-dihydro-3-(3-methylphenyl)-spiro[benzo(c)thiophen-1,4'-piperidin]-äthylenketal-maleinat vom Schmelzpunkt 155—157°C.

Analyse:

Berechnet für $C_{31}H_{34}FNO_2S.C_4H_4O_4$: 67,84%C; 6,18%H; 2,26%N.

Gefunden: 67,76%C; 6,19%H; 2,15%N.

Das Produkt dient zur weiteren Umsetzung.

Beispiel 47

Man versetzt 8,5 g Chlorameisensäurephenylester in 130 ml trockenem Dichlormethan unter Stickstoff im Verlauf von 15 Minuten mit der Lösung von 15,56 g der freien Base 3-(2,4-Dichlorbenzylthio)-1,3-dihydro-1'-methylspiro[benzo(c)thiophen-1,4'-piperidin] aus Beispiel 43 in 100 ml trockenem Dichlormethan. Man rührt 21 Stunden bei Raumtemperatur, verdünnt dann mit 450 ml Dichlormethan, wäscht mit zweimal je 300 ml 10%iger wässriger Natronlauge, zweimal je 300 ml Wasser und einmal 100 ml Salzlösung und trocknet über Magnesiumsulfat zu einem Oel. Das Oel wird über Silikagel mit Dichlormethan chromatographiert, was 3-(2,4-Dichlorphenyl)-1,3-dihydro-1'-phenoxycarbonylspiro[benzo(c)thiophen-1,4'-piperidin] als klares Oel ergibt.

Analyse:

Berechnet für $C_{25}H_{21}Cl_2NO_2S$: 63,84%C; 4,50%H; 2,98%N.

Gefunden: 63,71%C; 4,52%H; 2,79%N.

Das Produkt dient zur weiteren Umsetzung.

Beispiel 48

Man erhitzt die Lösung von 2,85 g der freien Base 1'-[3-(4-Fluorbenzoyl)-propyl]-1,3-dihydro-3-(2-methylphenyl)-spiro[benzo(c)thiophen-1,4'-piperidin]-äthylenketal aus Beispiel 38, 30 ml absolutem Aethanol und 30 ml 3n-HCl-Lösung 45 Minuten zum Rückfluss. Man kühlt ab, macht mit 40%iger wässriger Natronlauge alkalisch und extrahiert mit $CH_2Cl_2$. Die $CH_2Cl_2$-Fraktionen werden vereinigt, mit Wasser gewaschen und über $MgSO_4$ getrocknet. Die Lösung wird filtriert und zu einem Feststoff eingedampft, den man aus Essigester umkristallisiert, was 1'-[3-(4-Fluorbenzoyl)-propyl]-1,3-dihydro-3-(2-methylphenyl)-spiro[benzo(c)thiophen-1,4'-piperidin] als reines Produkt vom Schmelzpunkt 143—145°C ergibt.

Analyse:

Berechnet für $C_{29}H_{30}FNOS$: 75,78%C; 6,58%H; 3,05%N.

Gefunden: 75,51%C; 6,70%H; 2,85%N.

Beispiel 49

Man gibt 34,2 g 85%ige Kaliumhydroxydplätzchen unter Stickstoff bei 150°C zu 15,12 g 3-(2,4-Dichlorphenyl)-1,3-dihydro-1'-phenoxycarbonylspiro[benzo(c)thiophen-1,4'-piperidin] aus Beispiel 47 in 216 ml Aethylenglykol. Man rührt 45 Minuten bei 160°C, giesst dann in 1,4 1 Wasser und extrahiert mit dreimal je 350 ml Dichlormethan. Der vereinigte Dichlormethanextrakt wird mit zweimal je 400 ml Wasser und einmal 100 ml gesättigter wässriger Kochsalzlösung gewaschen und über Magnesiumsulfat zu einem Oel getrocknet. Dieses wird durch Abgiessen von dreimal je 225 ml siedendem Hexan gereinigt, was einen Feststoff liefert. Ein Teil des Feststoffs wird in Aether gelöst und zur Salzbildung mit ätherischer Maleinsäure behandelt. Das Salz wird mit Aether gewaschen und aus Methanol/Aceton/Aether zu 3-(2,4-Dichlorphenyl)-1,3-dihydrospiro[benzo(c)thiophen-1,4'-piperidin]-maleinat als weisses Pulver vom Schmelzpunkt 171—172,5°C umkristallisiert.

Analyse:

Berechnet für $C_{18}H_{17}Cl_2NS.C_4H_4O_4$: 56,66%C; 4,54%H; 3,00%N; 15,20%Cl.

Gefunden: 56,49%C; 4,54%H; 2,81%N; 14,99%Cl.

Beispiel 50

Man versetzt 3,0 g der freien Base 3-(2,4-Dichlorphenyl)-1,3-dihydrospiro[benzo(c)thiophen-1,4'-piperidin] aus Beispiel 49 in 35 ml siebgetrocknetem Dimethylformamid mit 2,62 g $\gamma$-Chlor-p-fluorbutyrophenon-äthylenketal und danach mit 1,7 g Natriumbicarbonat und 1,7 g Kaliumjodid. Man rührt 20 Stunden bei 80—90°C. Das Gemisch wird mit 75 ml Chloroform verdünnt, filtriert und am Rotationsverdampfer bei 75°C zu einem Oel eingedampft. Dieses wird zwischen 125 ml Dichlormethan und 85 ml Wasser verteilt. Man verdünnt das Gemisch mit 10 ml gesättigter wässriger

Natriumbicarbonatlösung, trennt die organische Schicht ab und wäscht einmal mit 80 ml Wasser und einmal mit 30 ml gesättigter Kochsalzlösung. Die Dichlormethanlösung wird getrocknet und zu einem Oel eingedampft. Man chromatographiert das Oel über Aluminiumoxyd mit Aether, gibt einen Teil des so erhaltenen Oels in Aether und behandelt zur Salzbildung mit ätherischer Oxalsäure.

Das Salz wird mit Aether gewaschen und aus Methanol/Aceton/Aether umkristallisiert, was 3-(2,4 - Dichlorphenyl) - 1' - [3 - (4 - fluorbenzoyl) - propyl] - 1,3 - dihydrospiro[benzo(c)thiophen - 1,4' - piperidin] - äthylenketal-oxalat als weisses Pulver vom Schmelzpunkt 164—165°C liefert.

Analyse:

Berechnet für $C_{30}H_{30}Cl_2FNO_2S.C_2H_2O_4$: 59,26%C; 4,97%H; 2,16%N.

Gefunden: 59,15%C; 4,81%H; 2,11%N.

Das Produkt dient zur weiteren Umsetzung.

Beispiel 51

a. Man gibt 33 ml Bortrifluoridätherat zu 26,33 g 4-(2-Fluorphenyl)-4-hydroxyl-1-methylpiperidin aus Beispiel 1a mit 20 ml (ungefähr 23,3 g) m-Fluorbenzylmercaptan in 25 ml Eisessig. Man rührt 44 Stunden bei 65—68°C, versetzt dann mit weiteren 17 ml Bortrifluoridätherat und rührt nochmals etwa drei weitere Tage bei 65—68°C. Ueberschüssiges Lösungsmittel wird unter vermindertem Druck bis 110°C entfernt. Den Rückstand vermischt man mit 450 ml 0,5n-HCl und 175 ml Aether, wobei sich ein Oel bildet. Das Oel und die Wasserschicht werden durch Abgiessen mit Aether gewaschen, und die wässrige Säure wird dann vorsichtig vom Oel abgegossen. Man trägt das Oel unter Wasser ein, stellt mit Ammoniumhydroxyd auf pH=9—10 und extrahiert mit Aether. Die ätherische Lösung wird mit Wasser neutral gewaschen und dann zur Salzbildung in eine auf 350 ml verdünnte Lösung von 30 ml 48%igem Bromwasserstoff gegossen. Das Salz wird abfiltriert und mit einer geringen Menge Wasser und reichlich Aether gewaschen, was 4-(3-Fluorbenzoylthio)-4-(2-fluorphenyl)-1-methylpiperidin-hydrobromid vom Schmelzpunkt 176—178,5°C als weisses Salz ergibt. Eine dreimal umkristallisierte Probe (Methanol/Aceton/Aether) schmilzt bei 178—179°C.

Analyse:

Berechnet für $C_{19}H_{21}F_2NS.HBr$: 55,08%C; 5,35%H; 3,38%N.

Gefunden: 55,05%C; 5,38%H; 3,38%N.

b. Unter Stickstoff gibt man 120 ml siebgetrocknetes Dimethylsulfoxyd zu 1,404 g Natriumhydrid (aus 2,87 g einer 50%igen Dispersion). Man rührt 35 Minuten in einem 80—85°C heissen Bad und kühlt dann auf Raumtemperatur ab. Man versetzt im Verlauf von 60 Sekunden mit der Lösung von 14,0 g der freien Base 4-(3-Fluorbenzylthio)-4-(2-fluorphenyl)-1-methylpiperidin aus Beispiel 51a in 47 ml DMSO und rührt eine Stunde. Der Ansatz wird dann in 700 ml Eiswasser gegossen und mit dreimal je 200 ml Dichlormethan extrahiert. Die vereinigten Dichlormethanextrakte wäscht man mit dreimal je 300 ml Wasser und einmal mit 75 ml gesättigter Kochsalzlösung und trocknet über Magnesiumsulfat zu einem Oel. Dieses wird über Aluminiumoxyd mit Aether chromatographiert, wobei sich Kristalle bilden. Ein Teil davon wird in Aether mit ätherischer Maleinsäure behandelt. Das so erhaltene Salz wäscht man mit Aether und trocknet, was 3-(3-Fluorphenyl)-1,3-dihydro-1'-methylspiro[benzo(c)thiophen-1,4'-piperidin]-maleinat als Pulver vom Schmelzpunkt 137—140°C ergibt. Ein zweimal umkristallisierter Anteil schmilzt bei 138,5—140,5°C. Bei fortgesetztem Erhitzen entwickelt sich eine neue Kristallform, die im Bereich 175—180°C zusammenfällt.

Analyse:

Berechnet für $C_{19}H_{20}FNS.C_4H_4O_4$: 64,32%C; 5,63%H; 3,26%N; 4,42%F.

Gefunden: 64,26%C; 5,58%H; 2,95%N; 4,58%F.

Beispiel 52

Man versetzt 3,26 g der freien Base 3-(2,4-Dichlorphenyl)-1'-[3-(4-fluorbenzoyl)-propyl]-1,3-dihydrospiro[benzo(c)thiophen-1,4'-piperidin]-äthylenketal aus Beispiel 50 in 30 ml warmem Aethanol langsam mit 30 ml 3n-Salzsäure. Man erhitzt 45 Minuten über einem Dampfbad. Man gibt weitere 10 ml Aethanol dazu und erhitzt nochmals 45 Minuten. Dann wird auf Raumtemperatur abgekühlt und mit 40%iger wässriger Natronlauge alkalisch gemacht. Man verdünnt mit 200 ml Wasser und extrahiert mit dreimal je 80 ml Dichlormethan. Die vereinigten Dichlormethanextrakte wäscht man mit zweimal je 100 ml Wasser und einmal 50 ml gesättigter wässriger Kochsalzlösung und trocknet über Magnesiumsulfat zu einem Oel. Dieses wird in Aether gelöst, über Celit filtriert und zur Salzbildung mit ätherischer Maleinsäure behandelt. Das Salz wird gut mit Aether gewaschen und aus Aceton/Aether umkristallisiert, was 3 - (2,4 - Dichlorphenyl) - 1' - [3 - (4 - fluorbenzoyl) - propyl] - 1,3 - dihydro - spiro[benzo(c)thiophen-1,4' - piperidin] - maleinat als weisses Pulver vom Schmelzpunkt 124—127°C ergibt. Eine dreimal umkristallisierte Probe schmilzt bei 127—129°C.

Analyse:

Berechnet für $C_{28}H_{26}Cl_2FNOS.C_4H_4O_4$: 60,96%C; 4,80%H; 2,22%N; 11,25%Cl.

Gefunden: 61,13%C; 4,78%H; 2,06%N, 11,02%Cl.

Beispiel 53

Man versetzt 5,0 g Chlorameisensäurephenylester in 80 ml Dichlormethan im Verlauf von 20

Minuten mit 7,89 g der freien Base 3-(3-Fluorphenyl)-1,3-dihydro-1′-methylspiro[benzo(c)thiophen-1,4′-piperidin] aus Beispiel 51 in 10 ml Dichlormethan. Man rührt 21 Stunden bei Raumtemperatur un verdünnt dann mit 200 ml Dichlormethan. Das so erhaltene Gemisch wird zweimal mit je 175 ml 10%iger wässriger Natronlauge, zweimal mit je 150 ml Wasser und einmal mit 70 ml gesättigter wässriger Kochsalzlösung gewaschen und über Magnesiumsulfat getrocknet, was ein Oel ergibt. Dieses chromatographiert man über Silikagel mit Dichlormethan, was ein Oel ergibt, welches beim Kristallisieren 3-(3-Fluorphenyl)-1,3-dihydro-1′-phenoxycarbonylspiro[benzo(c)thiophen-1,4′-piperidin] vom Schmelzpunkt 134—142°C liefert. Ein Teil wird zweimal aus Toluol/Hexan umkristallisiert, Schmelzpunkt 144—146°C.

Analyse:
Berechnet für $C_{25}H_{22}FNO_2S$: 71,58%C; 5,29%H; 3,34%N.
Gefunden: 71,60%C; 5,33%H; 3,22%N.

Das Produkt dient zur weiteren Umsetzung.

Beispiel 54

Man versetzt 6,97 g 3-(3-Fluorphenyl)-1,3-dihydro-1′-phenoxycarbonylspiro[benzo(c)thiophen-1,4′-piperidin] aus Beispiel 53 in 100 ml Aethylenglykol unter Stickstoff bei 150°C mit 16 g 85%igen Kaliumhydroxydplätzchen. Man rührt 45 Minuten in einem 160°C heissen Bad. Dann wird die Lösung in 225 ml Eiswasser gegossen, auf 500 ml verdünnt und dreimal mit je 175 ml Dichlormethan extrahiert. Die vereinigten Dichlormethanextrakte wäscht man mit je 200 ml Wasser und einmal mit 50 ml gesättigter wässriger Kochsalzlösung, trocknet über Magnesiumsulfat und dampft ein, was die feste freie Base ergibt. Diese wird in Aether zur Salzbildung mit ätherischer Maleinsäure behandelt. Man wäscht das Salz mit Aether und kristallisiert aus Methanol/Aceton/Aether um, was 3-(3-Fluorphenyl)-1,3-dihydrospiro[benzo(c)thiophen-1,4′-piperidin]-maleinat als weisses Pulver vom Schmelzpunkt 188—188,5°C ergibt.

Analyse:
Berechnet für $C_{18}H_{18}FNS.C_4H_4O_4$: 63,61%C; 5.34%H; 3.37%N; 4.57%F.
Gefunden: 63,53%C; 5.37%H; 3.31%N; 4,45%F.

Beispiel 55

Man versetzt 2,75 g (9,19 mMol) der freien Base 3-(3-Fluorphenyl)-1,3-dihydrospiro[benzo(c)-thiophen-1,4′-piperidin] aus Beispiel 54 in 47 ml siebgetrocknetem Dimethylformamid mit 2,81 g $\gamma$-Chlor-p-fluorbutyrophenon-äthylenketal sowie 1,82 g Kaliumjodid und 1,82 g Natriumbicarbonat. Man rührt 20 Stunden bei 65—90°C, verdünnt dann mit 75 ml Chloroform, filtriert und dampft am Rotationsverdampfer bei 75°C zu einem Oel ein. Dieses wird zwischen 125 ml Dichlormethan und 85 ml Wasser verteilt. Man gibt eine 10 ml-Portion gesättigte wässrige Natriumbicarbonatlösung dazu und scheidet die Lösungen. Die organische Schicht wird einmal mit 80 ml Wasser und einmal mit 30 ml gesättigter wässriger Kochsalzlösung gewaschen und über Magnesiumsulfat zu einem Oel getrocknet. Dieses chromatographiert man über Aluminiumoxyd mit Aether, was 3-(3-Fluorphenyl)-1′-[3-(4-fluorbenzoyl)-propyl]-1,3-dihydrospiro[benzo(c)thiophen-1,4′-piperidin]-äthylenketal ergibt. Umkristallisation aus Cyclohexan/Pentan liefert eine bei 121,5—123°C schmelzende Probe.

Analyse:
Berechnet für $C_{30}H_{31}F_2NO_2S$: 70,98%C; 6,17%H; 2,76%N.
Gefunden: 70,97%C; 6,20%H; 2,43%N.

Das Produkt dient zur weiteren Umsetzung.

Beispiel 56

Man versetzt 3,75 g 3-(3-Fluorphenyl)-1′-[3-(4-fluorbenzoyl)-propyl]-1,3-dihydrospiro[benzo(c)-thiophen-1,4′-piperidin]-äthylenketal aus Beispiel 55 in 38 ml Aethanol mit 38 ml 3n-Salzäure. Man erhitzt zwei Stunden unter Rückfluss und lässt 16 Stunden bei Raumtemperatur stehen. Man verdünnt die Lösung mit 100 ml Wasser und gibt einige ml 40%ige wässrige Natronlauge dazu, um den pH auf 11 zu stellen. Dann wird mit 175 ml Wasser verdünnt und dreimal mit je 175 ml Dichlormethan extrahiert. Der vereinigte Dichlormethanextrakt wird zweimal mit je 125 ml Wasser und einmal mit 60 ml gesättigter Kochsalzlösung gewaschen und über Magnesiumsulfat zu einem Oel getrocknet. Dieses behandelt man in Aether mit ätherischer Maleinsäure, wäscht das so erhaltene Salz mit Aether und kristallisiert aus Aceton/Aether um, was 1′-[3-(4-Fluorbenzoyl)-propyl]-3-(3-fluorphenyl)-1,3-dihydrospiro[benzo(c)thiophen-1,4′-piperidin]-maleinat als weisses kristallines Pulver vom Schmelzpunkt 149—150,5°C ergibt.

Analyse:
Berechnet für $C_{28}H_{27}F_2NOS.C_4H_4O_4$: 66,31%C; 5,39%H; 2,42%N; 6,56%F.
Gefunden: 66,35%C; 5,54%H, 2.14%N; 6,61%F.

Beispiel 57

Man versetzt 3,10 g der freien Base 1,3-Dihydro-3-(4-methylphenyl)-spiro[benzo(c)thiophen-1,4′-piperidin] aus Beispiel 36 in 54 ml Dimethylformamid mit 3,21 g $\gamma$-Chlor-p-fluorbutyrophenon-

äthylenketal, 2,07 g Kaliumjodid und 2,07 g Natriumbicarbonat. Das Reaktionsgemisch wird 18 Stunden gerührt und bei 65—90°C erhitzt. Man verdünnt mit 75 ml Chloroform, filtriert und dampft zu einem Oel ein. Dieses wird zwischen 150 ml Dichlormethan und 125 ml Wasser verteilt. Man wäscht die organische Schicht mit Wasser und wässriger Kochsalzlösung und trocknet dann über $MgSO_4$. Die Lösung wird filtriert und zu einem Oel eingedampft, das man über $Al_2O_3$/$Ät_2O$ säulenchromatographiert. Eine Probe der entstandenen freien Base wird in das Oxalatsalz von 1'-[3-(4-Fluorbenzoyl)-propyl]-3-(4-methylphenyl)-1,3-dihydrospiro[benzo(c)thiophen-1,4'-piperidin]-äthylenketal überführt.

Analyse:
Berechnet für $C_{31}H_{34}FNO_2S.C_2H_2O_4$: 66,76%C; 6,11%H, 2,36%N.
Gefunden: 66,85%C; 6,12%H, 2,32%N.

Das Produkt dient zur weiteren Umsetzung.

Beispiel 58

Man erhitzt die Lösung von 2,57 g 1'-[3-(4-Fluorbenzoyl)-propyl]-1,3-dihydro-3-(4-methylphenyl)-spiro[benzo(c)thiophen-1,4'-piperidin]-äthylenketal aus Beispiel 57, 27 ml absolutem Aethanol und 27 ml 3n-HCl 45 Minuten auf dem Dampfbad. Man kühlt ab, verdünnt mit 75 ml Wasser und stellt den pH mit 40%iger wässriger NaOH-Lauge auf 11. Die Lösung wird mit 130 ml Wasser verdünnt und mit Dichlormethan extrahiert. Die $CH_2Cl_2$-Fraktionen werden kombiniert, mit Wasser und Salzlösung gewaschen und über $MgSO_4$ getrocknet. Die getrocknete $CH_2Cl_2$-Lösung wird filtriert und zu einem Oel eingedampft, welches unter Pentan erstarrt. Das filtrierte produkt wird aus Essigester umkristallisiert, was 1' - [3 - (4 - Fluorbenzoyl) - propyl] - 1,3 - dihydro - 3 - (4 - methylphenyl) - spiro[benzo(c)thiophen - 1,4' - piperidin] vom Schmelzpunkt 134—135°C ergibt.

Analyse:
Berechnet für $C_{29}H_{30}FNOS$: 75,78%C; 6,58%H; 3,05%N.
Gefunden: 75,61%C; 6,67%H; 3,07%N.

Beispiel 59

Man versetzt 6,87 g in 33 ml 2-Butanon sowie 33 ml Dimethylformamid gelöstes 3-(4-Chlorphenyl)-1,3-dihydrospiro[benzo(c)thiophen-1,4'-piperidin] aus Beispiel 13 mit 6,04 g $\gamma$-Chlor-2,4-difluorbutyrophenon-äthylenketal und danach mit 6,5 g Natriumbicarbonat und 4,08 g Kaliumjodid. Man rührt 18 Stunden bei 75—80°C, gibt dann 400 ml Eiswasser dazu und extrahiert dreimal mit je 125 ml Aether. Die vereinigten Aetherextrakte werden zweimal mit je 150 ml Wasser und einmal mit 30 ml Salzlösung gewaschen und über Magnesiumsulfat getrocknet, was ein Oel liefert. Dieses chromatographiert man über 300 ml Aluminiumoxyd mit Aether, wobei man ein Oel erhält, das schliesslich zu einem produkt kristallisiert. Ein Teil des Produkts wird mit Aether verdünnt und zur Salzbildung mit ätherischer Maleinsäure behandelt. Das Salz wird einmal aus Methanol/Aether und einmal aus Aceton/Essigester/Pentan umkristallisiert, was 3-(4-Chlorphenyl)-1'-[3-(2,4-difluorbenzoyl)-propyl]-1,3-dihydrospiro[benzo(c)thiophen-1,4'-piperidin)]-äthylenketal-maleinat vom Schmelzpunkt 148—149°C ergibt.

Analyse:
Berechnet für $C_{30}H_{30}ClF_2NO_2S.C_4H_4O_4$: 62,05%C; 5,21%H; 2,13%N.
Gefunden: 61,94%C; 5,01%H, 2,16%N.

Das Produkte dient zur weiteren Umsetzung.

Beispiel 60

Man versetzt 8,21 g (15,15 mMol) der freien Base 3-(4-Chlorphenyl)-1-[3-(2,4-difluorbenzoyl)-propyl]-1,3-dihydrospiro[benzo(c)thiophen-1,4'-piperidin]-äthylenketal aus Beispiel 59 in 62 ml 95%igem warmen Aethanol langsam mit 31 ml 3n-Salzsäure, wobei eine Suspension entsteht. Man rührt 3 Stunden bei Raumtemperatur und 45 Minuten auf dem Dampfbad. Dann wird mit 400 ml Wasser verdünnt und mit Ammoniumhydroxyd alkalisch gemacht. Das wässrige Gemisch extrahiert man zweimal mit je 150 ml Aether und zweimal mit je 250 ml Aether/Toluol 1:1. Die vereinigten organischen Schichten werden mit Wasser und gesättigter wässriger Kochsalzlösung gewaschen und über Magnesiumsulfat zu einem braunen Oel getrocknet. Dieses chromatographiert man über Silikagel mit Aether, was einen Feststoff ergibt. Ein Teil des Produkts wird dreimal aus Aether/Pentan umkristallisiert, was 3-(4 - Chlorphenyl) - 1' - [3 - (2,4 - difluorbenzoyl) - propyl] - 1,3 - dihydrospiro[benzo(c)thiophen - 1,4' - piperidin] vom Schmelzpunkt 98—100°C ergibt.

Analyse:
Berechnet für $C_{28}H_{26}ClF_2NOS$: 67,52%C; 5,26%H, 2,81%N.
Gefunden: 67,25%C; 5,49%H; 2,69%N.

Beispiel 61

Man erhitzt ein Gemisch aus 1,3-Dihydro-3-phenylspiro[benzo(c)thiophen-1,4'-piperidin] aus Beispiel 4 (3,75 g), 3,7 g $\gamma$-Chlor-2,4-difluorbutyrophenon-äthylenketal, 2,5 g KJ und 4,0 g Natriumbicarbonat in einem Gemisch aus 20 ml DMF und 20 ml 2-Butanon 3 Stunden zum Rückfluss. Nach Abkühlung verdünnt man mit Wasser, extrahiert dreimal mit Aether und engt die gewaschene und getrocknete Aetherlösung zu einem öligen Rückstand ein. Dieser wird gereinigt, indem man ihn durch eine Aluminiumoxydsäule laufen lässt. Elution mit Aether liefert das tertiäre Amin, welches man in das kristalline Maleinat, Schmelzpunkt 171—172,5°C, von 1′-[3-(2,4-Difluorbenzoyl)-propyl]-1,3-dihydro-3-phenylspiro[benzo(c)thiophen-1,4′-piperidin]-äthylenketal überführt.

Analyse:
Berechnet für $C_{30}H_{31}F_2NO_2S.C_4H_4O_4$: 65,47%C; 5,66%H; 5,14%S.
Gefunden: 65,21%C; 5,68%H; 5,36%S.

Das Produkt dient zur weiteren Umsetzung.

Beispiel 62

Man lässt die Lösung von 5,0 g der freien Base 1′-[3-(2,4-Difluorbenzoyl)-propyl]-1,3-dihydro-3-phenylspiro[benzo(c)thiophen-1,4′-piperidin]-äthylenketal aus Beispiel 61 in 20 ml 3n-HCl und 20 ml Wasser 3 Stunden bei Raumteperatur stehen. Der Ueberschuss Säure und Lösungsmittel wird unter vermindertem Druck bei 60°C entfernt und der Rückstand mit konzentriertem $NH_4OH$ alkalisch gemacht. Das freigesetzte Oel nimmt man in Aether auf, wäscht und trocknet. Entfernung der Lösungsmittel liefert ein klares Oel, welches man in Aether in das kristalline, Maleinat, Schmelzpunkt 134—135°C, von 1′-[3-(2,4-Difluorbenzoyl)-propyl]-1,3-dihydro-3-phenylspiro[benzo(c)thiophen-1,4′-piperidin] überführt.

Analyse:
Berechnet für $C_{28}H_{27}F_2NOS.C_4H_4O_4$: 66,67%C; 5,39%H; 5,53%S.
Gefunden: 66,21%C; 5,36%H; 5,79%S.

Beispiel 63

Man versetzt 5,60 g der freien Base 1′-[3-(4-Fluorbenzoyl)-propyl]-3-(3-methylphenyl)-1,3-dihydrospiro[benzo(c)thiophen-1,4′-piperidin]-äthylenketal aus Beispiel 46 in 56 ml warmem Aethanol langsam mit 56 ml 3n-Salzsäure. Man erhitzt 45 Minuten zum Rückfluss, kühlt dann auf Raumtemperatur ab, macht mit einer kleinen Menge 40%iger wässriger Natronlauge alkalisch und verdünnt mit 350 ml Wasser. Die Lösung wird dreimal mit je 150 ml Dichlormethan extrahiert. Der vereinigte Dichlormethanextrakt wird mit zweimal je 200 ml Wasser und einmal 75 ml Salzlösung gewaschen und über Magnesiumsulfat getrocknet, was ein Oel ergibt. Dieses löst man in Aether, filtriert und behandelt zur Salzbildung mit ätherischem Bromwasserstoff. Das Salz wird mit Aether gewaschen und aus Methanol/Aceton/Aether umkristallisiert, was 1′-[3-(4-Fluorbenzoyl)-propyl]-1,3-dihydro-3-(3-methylphenyl)-spiro[benzo(c)thiophen-1,4′-piperidin]-hydrobromid als Pulver ergibt, das bei 190—195°C zu einem Gel zusammenschrumpft und bei 217—218°C zu einer dunklen Flüssigkeit schmilzt.

Analyse:
Berechnet für $C_{29}H_{30}FNOS.HBr$: 64,44%C; 5,78%H; 2,59%N; 3,52%F.
Gefunden: 64,30%C; 5,79%H; 2,48%N; 3,56%F.

**Patentansprüche**

1. Substituierte 1,3-Dihydrospiro[benzo(c)thiophene) der Formel

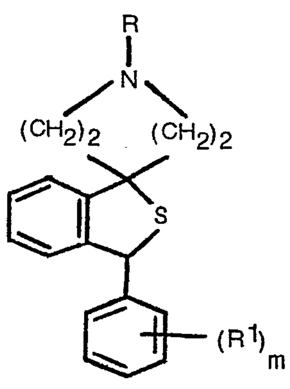

I

oder deren optische Antipoden bzw. pharmazeutisch unbedenklichen Salze, worin R für Wasserstoff, $C_{1-6}$-Alkyl, $C_{3-6}$-Cycloalkyl-$C_{1-6}$-alkyl oder Benzoyl-$C_{1-6}$-alkyl, wobei der Phenylrest mit Fluor substituiert sein kann, $R^1$ für Wasserstoff, $C_{1-6}$-Alkyl oder Halogen steht und m 1 oder 2 bedeutet.

2. Verbindungen nach Anspruch 1, dadurch gekennzeichnet, daß R für Wasserstoff, Methyl, Cyclopropylmethyl oder 4-Fluorbenzoylpropyl steht.

3. Verbindungen gemäß Anspruch 1 zur Verwendung als Arzneimittel.

4. Verfahren zur Herstellung von Verbindungen der Formel I gemäß Anspruch 1, dadurch gekennzeichnet, daß man

a) ein 2-Bromfluorbenzol der Formel

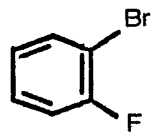

in einem geeigneten Lösungsmittel mit einem Niederalkyllithium in sein 2-Lithioderivat überführt,

b) das 2-Lithioderivat mit einem Cycloazalkanon der Formel

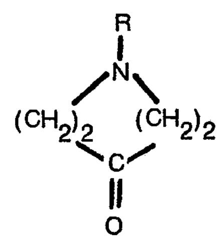

worin R für $C_{1-6}$-Alkyl steht, zu einem Phenylcycloazalkanol der Formel

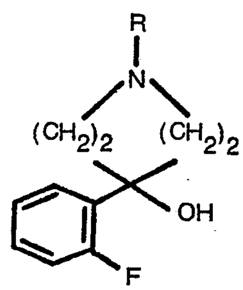

umsetzt,

c) das Phenylcycloazalkanol in Gegenwart einer Lewissäure mit einem Benzylmercaptan de Formel

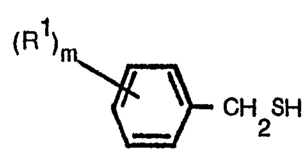

zu einem 4-Benzylthio-4-phenylcycloazalkan der Formel

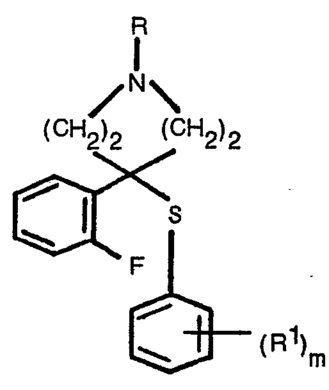

umsetzt,

d) das 4-Benzylthio-4-phenylcycloazalkan zum Ring schließt,

e) gegebenenfalls eine Verbindung der Formel I, worin R für $C_{1-6}$-Alkyl steht, in einem geeigneten Lösungsmittel mit einem Chlorameisensäure-$C_{1-6}$-alkylester oder Chlorameisensäurephenylester umsetzt zu einer Verbindung der Formel

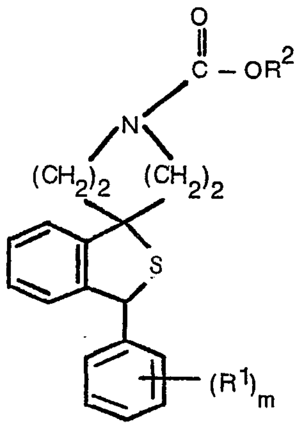

worin $R^2$ Phenyl oder $C_{1-6}$-Alkyl bedeutet, und die erhaltene Verbindung mit einer Base oder einer Säure behandelt, was zu einer Verbindung der Formel I, worin R für Wasserstoff steht, führt,

f) gegebenenfalls eine Verbindung der Formel I, worin R für Wasserstoff steht, mit einer Verbindung der Formel

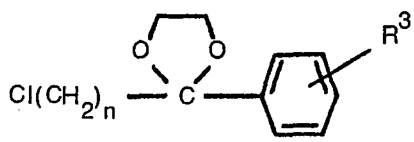

worin $R^3$ Wasserstoff oder Fluor bedeutet, und n eine Zahl von 1—6 darstellt, umsetzt zu einer Verbindung der Formel I, worin R den Rest

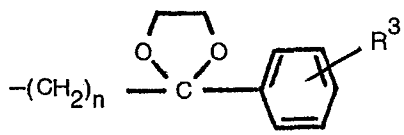

bedeutet, und $R^1$, $R^3$, m und n die oben angegebene Bedeutung haben, und die erhaltene Verbindung hydrolysiert zu einer Verbindung der Formel

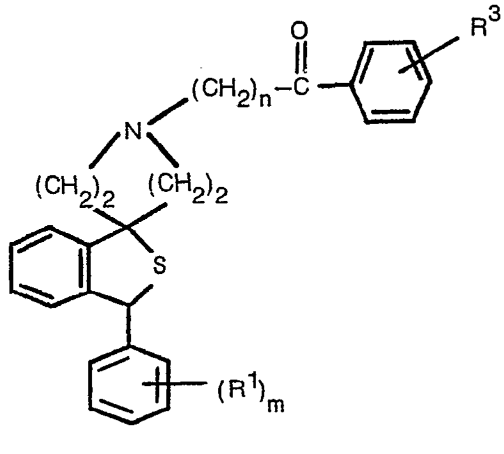

worin $R^1$, $R^3$, m und n die oben angegebene Bedeutung haben, und

g) gegebenenfalls eine Verbindung der Formel I, worin R Wasserstoff bedeutet, mit einem $C_{1-6}$-Alkanoylchlorid oder -anhydrid, $C_{1-6}$-Alkylhalogenid, $C_{3-6}$-Cycloalkyl-$C_{1-6}$-alkylhalogenid, $C_{3-6}$-Cycloalkyl-$C_{1-6}$-alkanoylhalogenid zu einer Verbindung der Formel I umsetzt, worin $R^1$ und m die oben angegebene Bedeutung haben und R $C_{1-6}$-Alkanoyl, $C_{1-6}$-Alkyl, $C_{3-6}$-Cycloalkyl-$C_{1-6}$-alkyl oder $C_{3-6}$-Cycloalkyl-$C_{1-6}$-alkanoyl bedeutet, und

h) gegebenenfalls eine Verbindung der Formel I, worin $R^1$ und m die oben angegebene Bedeutung haben und R für $C_{2-6}$-Alkoxycarbonyl, $C_{1-6}$-Alkanoyl oder $C_{3-6}$-Cycloalkyl-$C_{1-6}$-alkanoyl steht, zu einer Verbindung der Formel I reduziert, worin R $C_{1-6}$-Alkyl oder $C_{3-6}$-Cycloalkyl-$C_{1-6}$-alkyl bedeutet.

**Revendications**

1. 1,3-dihydrospiro[benzo(c)thiophènes] substitués répondant à la formule:

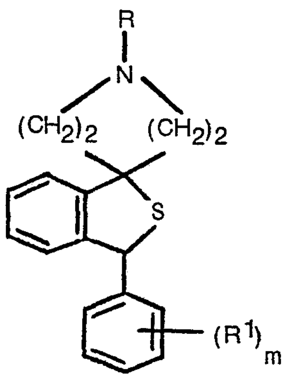

I

dans laquelle R représente l'hydrogène, un groupe alcoyle en $C_{1-6}$, cycloalcoyl($C_{3-6}$)alcoyle en $C_{1-6}$ ou benzoyl-alcoyle en $C_{1-6}$, auquel cas le radical phényle peut être substitué par le fluor, R' représente l'hydrogène, un groupe alcoyle en $C_{1-6}$ ou un halogène et m est 1 ou 2, ainsi que leurs antipodes optiques et leurs sels pharmaceutiquement acceptables.

2. Composés selon la revendication 1, caractérisés en ce que R représente l'hydrogène, un groupe méthyle, cyclopropylméthyle ou 4-fluorobenzoylpropyle.

3. Composés selon la revendication 1, destinés à être utilisés comme médicaments.

4. Procédé pour la préparation de composés de formule I selon la revendication 1, caractérisé en ce que:

a) on convertit un 2-bromofluorobenzène de formule:

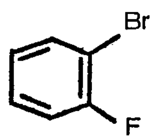

dans un solvant approprié en son dérivé 2-lithio avec un alcoyl lithium inférieur,

b) on fait réagir le dérivé 2-lithio avec une cycloazalcanone de formule:

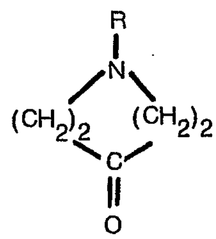

dans laquelle R représente un groupe alcoyle en $C_{1-6}$, pour obtenir un phénylcycloazalcanol de formule:

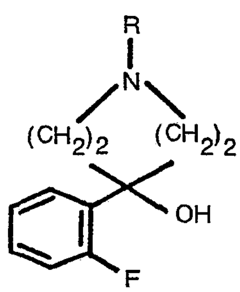

c) on fait réagir le phénylcycloazalcanol en présence d'un acide de Lewis avec un benzyl mercaptan de formule:

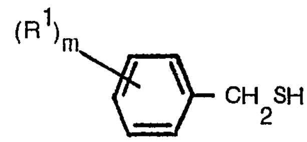

pour obtenir un 4-benzylthio-4-phénylcycloazalcane de formule:

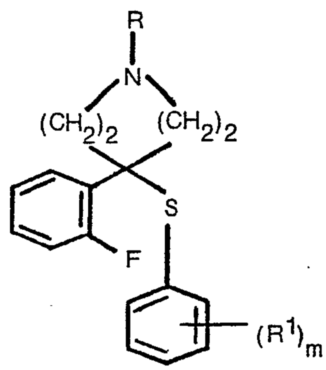

d) on cyclise le 4-benzylthio-4-phénylcycloazalcane,

e) éventuellement on fait réagir un composé de formule I, dans lequel R représente un groupe alcoyle en $C_{1-6}$, dans un solvant approprié, avec un ester d'alcoyle en $C_{1-6}$ de l'acide chloroformique, ou l'ester phénylique de l'acide chloroformique, pour obtenir un composé de formule:

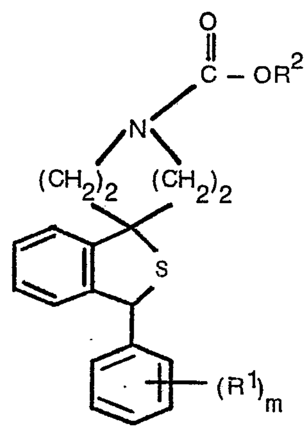

dans laquelle $R^2$ représente un groupe phényle ou alcoyle en $C_{1-6}$, et on traite le composé obtenu avec une base ou un acide pour obtenir un composé de formule I, dans lequel R représente l'hydrogène;

f) éventuellement on fait réagir un composé de formule I, dans lequel R représente l'hydrogène, avec un composé de formule:

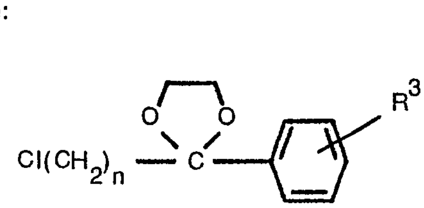

dans laquelle $R^3$ représente l'hydrogène ou le fluor, et n est un nombre compris entre 1 et 6, pour obtenir un composé de formule I, dans lequel R représente le radical:

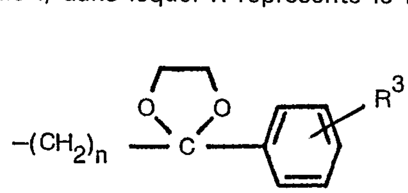

et $R^1$, $R^3$, m et n ont les significations indiquées ci-dessus, et on hydrolyse le composé obtenu en un composé de formule:

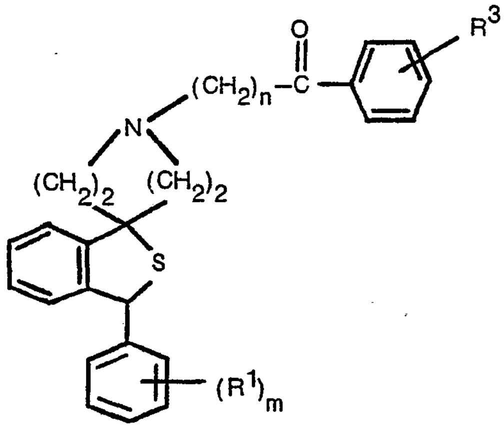

dans laquelle $R^1$, $R^3$, m et n ont les significations indiquées, ci-dessus, et

g) éventuellement on fait réagir un composé de formule I, dans lequel R représente l'hydrogène, avec un chlorure ou un anhydride d'alcanoyle en $C_{1-6}$, un halogénure d'alcoyle en $C_{1-6}$, un halogénure de cycloalcoyl($C_{3-6}$)alcoyle en $C_{1-6}$, un halogénure de cycloalcoyl($C_{3-6}$)-alcanoyle, en $C_{1-6}$, pour obtenir un composé de formule I, dans lequel $R^1$ et m ont les significations indiquées ci-dessus et R représente un groupe alcanoyle en $C_{1-6}$, alcoyle en $C_{1-6}$, cycloalcoyl($C_{3-6}$)-alcoyle en $C_{1-6}$ ou cycloalcoyl($C_{3-6}$)-alcanoyle en $C_{1-6}$, et

h) éventuellement on réduit un composé de formule I, dans lequel $R^1$ et m ont les significations ci-dessus et R représente un groupe alcoxy($C_{2-6}$)-carbonyle, alcanoyle en $C_{1-6}$ ou cycloalcoyl($C_{3-6}$)-alcanoyle en $C_{1-6}$, en un composé de formule I, dans lequel R représente un groupe alcoyle en $C_{1-6}$ ou cycloalcoyl($C_{3-6}$)-alcoyle en $C_{1-6}$.

**Claims**

1. Substituted 1,3-Dihydrospiro[benzo(c)thiophene] of the formula

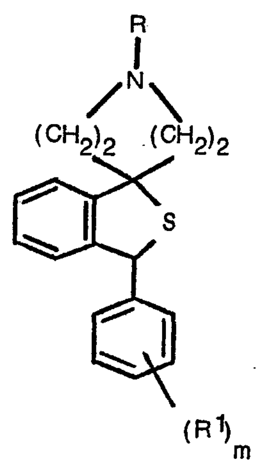

I

or an optical antipode or pharmaceutically acceptable salt thereof in which R is hydrogen, $C_1$—$C_6$-alkyl, $C_3$—$C_6$-cycloalkyl-$C_1$—$C_6$-alkyl or benzoyl-$C_1$—$C_6$-alkyl, wherein the phenyl radical may be substituted with fluorine, $R^1$ is hydrogen, $C_1$—$C_6$-alkyl or halogen and m is the integer 1 or 2.

2. A compound defined in claim 1 in which R is hydrogen, methyl, cyclopropylmethyl or 4-fluorobenzoylpropyl.

3. A compound as claimed in claim 1 for use as a medicament.

4. A process for preparing a compound of the formula I as defined in claim 1, which comprises

a) converting a 2-bromo-fluorobenzene of the formula

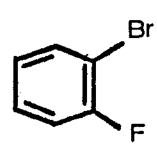

to its 2-lithio derivative with a lower alkyllithium in a suitable solvent

b) reacting the 2-lithio derivative with a cycloazalkanone of the formula

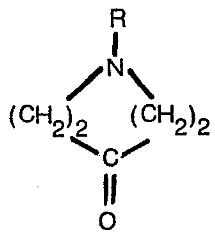

in which R is $C_1$—$C_6$-alkyl to provide a phenylcycloazalkanol of the formula

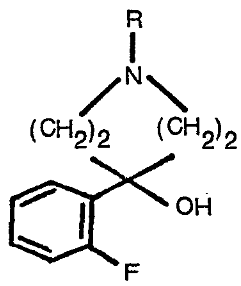

c) reacting the phenylcycloazalkanol with a benzylmercaptane of the formula

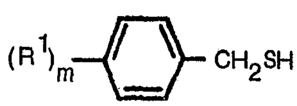

in the presence of a Lewis Acid to provide a 4-benzylthio-4-phenylcycloazalkane of the formula

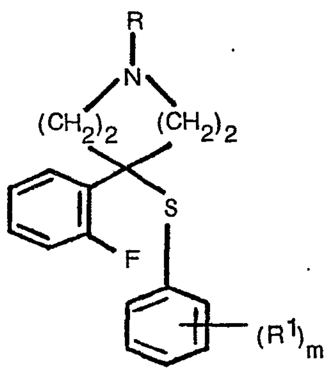

d) cyclizing the 4-benzylthio-4-phenylcycloazalkane,

e) optionally reacting a compound of the formula I in which R is $C_1$—$C_6$-alkyl in a suitable solvent with an alkyl chloroformic acid $C_1$—$C_6$ alkylester or phenyl chloroformate to provide a compound of the formula

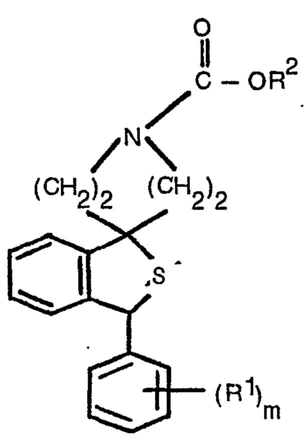

in which $R^2$ is phenyl or $C_1$—$C_6$-alkyl, and treating the compound obtained with a base or an acid, to provide a compound of the formula I in which R is hydrogen,

f) optionally treating a compound of the formula I in which R is hydrogen with a compound of the formula

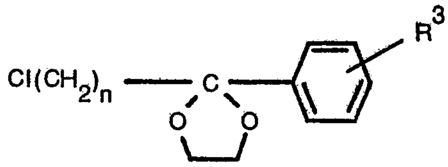

in which $R^3$ is hydrogen or fluorine and n is an integer from 1 to 6 to provide a compound of the formula I wherein R is the group

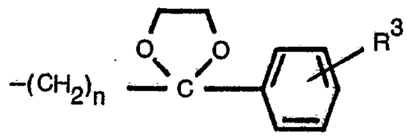

and $R^1$, $R^3$, m and n are as previously defined and hydrolyzing the compound obtained to provide a compound of the formula

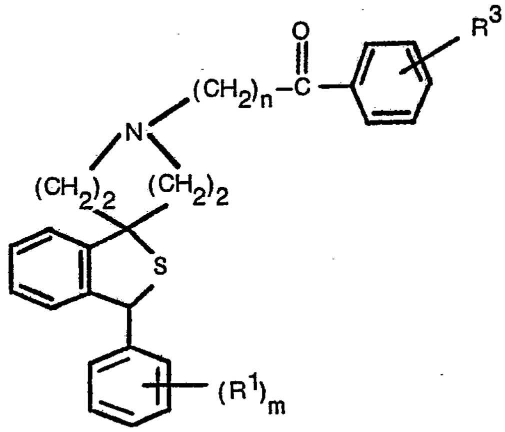

in which $R^1$, $R^3$, m and n are as defined above, and

g) optionally reacting a compound of the formula I in which R is hydrogen with a $C_1$—$C_6$-alkanoyl chloride or anhydride, $C_1$—$C_6$-alkyl halide, $C_3$—$C_6$-cycloalkyl-$C_1$—$C_6$-alkyl halide, $C_3$—$C_6$-cycloalkyl-$C_1$—$C_6$-alkanoyl halide to provide a compound of the formula I in which $R^1$ and m are as defined above and R is $C_1$—$C_6$-alkanoyl, $C_1$—$C_6$-alkyl, $C_3$—$C_6$-cycloalkyl-$C_1$—$C_6$-alkyl or $C_3$—$C_6$-cycloalkyl-$C_1$—$C_6$-alkanoyl, and

h) optionally reducing a compound of the formula I wherein $R^1$ and m are as defined above and R is $C_2$—$C_6$-alkoxycarbonyl, $C_1$—$C_6$-alkanoyl or $C_3$—$C_6$-cycloalkyl-$C_1$—$C_6$-alkanoyl to provide a compound of the formula I wherein R is $C_1$—$C_6$-alkyl or $C_3$—$C_6$-cycloalkyl-$C_1$—$C_6$-alkyl.